# EUROPEAN PATENT SPECIFICATION

(11) **EP 1 404 869 B1**
(45) Date of publication and mention of the grant of the patent: **05.08.2009**
(21) Application number: 02714330.4
(22) Date of filing: 20.03.2002
(51) Int. Cl.: C12Q 1/68

(54) **DETECTION OF MICROORGANISMS USING INDUCIBLE GENES**
NACHWEIS VON MIKROORGANISMEN MITTELS INDUZIERBARER GENE
DETECTION DE MICRO-ORGANISMES UTILISANT DES GENES INDUCTIBLES

(30) Priority: 20.03.2001 GB 0106949
(43) Date of publication of application: 07.04.2004
(73) Proprietor: Norchip A/S, 3490 Klokkarstua (NO)
(72) Inventor: KARLSEN, Frank, N-3490 Klokkarstua (NO)
(74) Representative: Murphy, Colm Damien
(86) International application number: PCT/GB2002/001308
(87) International publication number: WO 2002/074991

(56) References cited:
- EP-A- 0 517 154
- EP-A- 0 592 894
- WO-A-93/16172
- WO-A-98/25947
- WO-A-99/47705
- US-A- 5 525 463
- US-A- 5 563 033
- US-A- 5 656 424
- ALLAND ET AL.: "Identification of differentially expressed mRNA in prokaryotic organisms by customized amplification libraries (DECAL): the effect of isoniazid on gene expression in Mycobacterium tuberculosis" PNAS USA, vol. 95, December 1998 (1998-12), pages 13227-13232, XP002236208
- VAN DER VLIET ET AL.: "Nucleic acid sequence-based amplification (NASBA) for the identification of mycobacteria" J. GEN MICROBIOL, vol. 139, 1993, pages 2423-2429, XP000601914
- ESCUYER ET AL.: "Molecular characterization of a surface-exposed superoxide dismutase of Mycobacterium avium" MICROBIAL PATHOGENESIS, vol. 20, 1996, pages 41-55, XP002236210
- WHITEHOUSE C A ET AL.: "Identification of superoxide dismutase activity in Borrelia burgdorferi" INFECTION AND IMMUNITY, vol. 65, 1997, pages 4865-4868,
- SCHMIDT B L: "PCR in laboratory diagnosis of human Borrelia burgdorferi infections" CLINICAL MICROBIOLOGY REVIEWS, vol. 10, 1997, pages 185-201,

### SEQUENCE LISTING

<110> NORCHIP A/S
<120> DETECTION OF INDUCIBLE GENES IN MICROORGANISMS
<130> SJA/56821/001
<160> 99
<170> PatentIn version 3.1
<210> 1
   <211> 20
   <212> DNA
   <213> Artificial sequence
<220>
   <223> primer
<400> 1
   gaagcggcgg actaccatca 20
<210> 2
   <211> 20
   <212> DNA
   <213> Artificial sequence
<220>
   <223> primer
<400> 2
   ctcgattgcc gacgtgtcca 20
<210> 3
   <211> 20
   <212> DNA
   <213> Artificial sequence
<220>
   <223> probe
<400> 3
   cattgcgtca gcggtactcc 20
<210> 4
   <211> 32
   <212> DNA
   <213> Artificial sequence
<220>
   <223> molecular beacon probe
<400> 4
   cccgatcatt gcgtcagcgg tactccatcg gg 32
<210> 5
   <211> 34
   <212> DNA
   <213> Artificial sequence
<220>
   <223> molecular beacon probe
<400> 5
   gcccgatcat tgcgtcagcg gtactccatc gggc 34
<210> 6
   <211> 32
   <212> DNA
   <213> Artificial sequence
<220>
   <223> molecular beacon probe
<400> 6
   ccgccgcatt gcgtcagcgg tactcccggc gg 32
<210> 7
   <211> 20
   <212> DNA
   <213> Artificial sequence
<220>
   <223> primer
<400> 7
   accaaggact tccacatcga 20
<210> 8
   <211> 20
   <212> DNA
   <213> Artificial sequence
<220>
   <223> primer
<400> 8
   taccgaccac atcgacctca 20
<210> 9
   <211> 20
   <212> DNA
   <213> Artificial sequence
<220>
   <223> probe
<400> 9
   attgcgtcag cggtactccc 20
<210> 10
   <211> 32
   <212> DNA
   <213> Artificial sequence
<220>
   <223> molecular beacon probe
<400> 10
   cgtcgtattg cgtcagcggt actcccacga cg 32
<210> 11
   <211> 32
   <212> DNA
   <213> Artificial sequence
<220>
   <223> molecular beacon probe
<400> 11
   cgtcggattg cgtcagcggt actcccccga cg 32
<210> 12
   <211> 32
   <212> DNA
   <213> Artificial sequence
<220>
   <223> molecular beacon probe
<400> 12
   aggcgtattg cgtcagcggt actcccacgc ct 32
<210> 13
   <211> 20
   <212> DNA
   <213> Artificial sequence
<220>
   <223> primer
<400> 13
   gtgaccactt ctccggcaca 20
<210> 14
   <211> 20
   <212> DNA
   <213> Artificial sequence
<220>
   <223> primer
<400> 14
   gtgtaggcac ccttgtagaa 20
<210> 15
   <211> 20
   <212> DNA
   <213> Artificial sequence
<220>
   <223> probe
<400> 15
   gactattcct cgtcgtggcc 20
<210> 16
   <211> 32
   <212> DNA
   <213> Artificial sequence
<220>
   <223> molecular beacon probe
<400> 16
   cgcatggact attcctcgtc gtggcccatg cg 32
<210> 17
   <211> 30
   <212> DNA
   <213> Artificial sequence
<220>
   <223> molecular beacon probe
<400> 17
   ccagcgacta ttcctcgtcg tggccgctgg 30
<210> 18
   <211> 30
   <212> DNA
   <213> Artificial sequence
<220>
   <223> molecular beacon probe
<400> 18
   tcgcggacta ttcctcgtcg tggcccgcga 30
<210> 19
   <211> 20
   <212> DNA
   <213> Artificial sequence
<220>
   <223> primer
<400> 19
   ctgttttggg cgtcgatcca 20
<210> 20
   <211> 20
   <212> DNA
   <213> Artificial sequence
<220>
   <223> primer
<400> 20
   cagttggcgg ggacggctaa 20
<210> 21
   <211> 20
   <212> DNA
   <213> Artificial sequence
<220>
   <223> probe.
<400> 21
   attcccacta ccacgtccgg 20
<210> 22
   <211> 32
   <212> DNA
   <213> Artificial sequence
<220>
   <223> molecular beacon probe
<400> 22
   cgcatgattc ccactaccac gtccggcatg cg 32
<210> 23
   <211> 32
   <212> DNA
   <213> Artificial sequence
<220>
   <223> molecular beacon probe
<400> 23
   cctgcgattc ccactaccac gtccggcgca gg 32
<210> 24
   <211> 30
   <212> DNA
   <213> Artificial sequence
<220>
   <223> molecular beacon probe
<400> 24
   gcgctattcc cactaccacg tccggagcgc 30
<210> 25
   <211> 20
   <212> DNA
   <213> Artificial sequence
<220>
   <223> primer
<400> 25
   ggcgtcgatc cactgcggga 20
<210> 26
   <211> 20
   <212> DNA
   <213> Artificial sequence
<220>
   <223> primer
<400> 26
   ggtgggcaag aagaagtcga 20
<210> 27
   <211> 20
   <212> DNA
   <213> Artificial sequence
<220>
   <223> probe
<400> 27
   gcccgctcca gcacctcctt 20
<210> 28
   <211> 32
   <212> DNA
   <213> Artificial sequence
<220>
   <223> molecular beacon probe
<400> 28
   cgcatggccc gctccagcac ctccttcatg cg 32
<210> 29
   <211> 32
   <212> DNA
   <213> Artificial sequence
<220>
   <223> molecular beacon probe
<400> 29
   ccgtcggccc gctccagcac ctccttcgac gg 32
<210> 30
   <211> 30
   <212> DNA
   <213> Artificial sequence
<220>
   <223> molecular beacons probe
<400> 30
   cacgcgcccg ctccagcacc tccttgcgtg 30
<210> 31
   <211> 20
   <212> DNA
   <213> Artificial sequence
<220>
   <223> primer
<400> 31
   ctccagcacc tccttggtca 20
<210> 32
   <211> 20
   <212> DNA
   <213> Artificial sequence
<220>
   <223> primer
<400> 32
   ccaggctgat accacgtcta 20
<210> 33
   <211> 20
   <212> DNA
   <213> Artificial sequence
<220>
   <223> probe
<400> 33
   acttcttctt gcccacccac 20
<210> 34
   <211> 32
   <212> DNA
   <213> Artificial sequence
<220>
   <223> molecular beacon probe
<400> 34
   cgcatgactt cttcttgccc acccaccatg cg 32
<210> 35
   <211> 32
   <212> DNA
   <213> Artificial sequence
<220>
   <223> molecular beacon probe
<400> 35
   ccgtcgactt cttcttgccc acccaccgac gg 32
<210> 36
   <211> 30
   <212> DNA
   <213> Artificial sequence
<220>
   <223> molecular beacon probe
<400> 36
   cacgcacttc ttcttgccca cccacgcgtg 30
<210> 37
   <211> 20
   <212> DNA
   <213> Artificial sequence
<220>
   <223> primer
<400> 37
   cctccttggt caattcgtta 20
<210> 38
   <211> 20
   <212> DNA
   <213> Artificial sequence
<220>
   <223> primer
<400> 38 20
   aagtcaaccc tgctccatga 20
<210> 39
   <211> 20
   <212> DNA
   <213> Artificial sequence
<220>
   <223> probe
<400> 39
   ttgcccaccc accccataga 20
<210> 40
   <211> 32
   <212> DNA
   <213> Artificial sequence
<220>
   <223> molecular beacon probe
<400> 40
   cgcatgttgc ccacccaccc catagacatg cg 32
<210> 41
   <211> 30
   <212> DNA
   <213> Artificial sequence
<220>
   <223> molecular beacon probe
<400> 41
   cgctgttgcc cacccacccc atagacagcg 30
<210> 42
   <211> 30
   <212> DNA
   <213> Artificial sequence
<220>
   <223> molecular beacon probe
<400> 42
   cgcatttgcc cacccacccc atagaatgcg 30
<210> 43
   <211> 40
   <212> DNA
   <213> Artificial sequence
<220>
   <223> primer
<400> 43
   gatgcaaggt cgcatatgag gaagcggcgg actaccatca 40
<210> 44
   <211> 51
   <212> DNA
   <213> Artificial sequence
<220>
   <223> primer
<400> 44
   aattctaata cgactcacta tagggagaag gctcgattgc cgacgtgtcc a 51
<210> 45
   <211> 40
   <212> DNA
   <213> Artificial sequence
<220>
   <223> primer
<400> 45
   gatgcaaggt cgcatatgag accaaggact tccacatcga 90
<210> 46
   <211> 51
   <212> DNA
   <213> Artificial sequence
<220>
   <223> primer
<400> 46
   aattctaata cgactcacta tagggagaag gtaccgacca catcgacctc a 51
<210> 47
   <211> 40
   <212> DNA
   <213> Artificial sequence
<220>
   <223> primer
<400> 47
   gatgcaaggt cgcatatgag gtgaccactt ctccggcaca 40
<210> 48
   <211> 51
   <212> DNA
   <213> Artificial sequence
<220>
   <223> primer
<400> 48
   aattctaata cgactcacta tagggagaag ggtgtaggca cccttgtaga a 51
<210> 49
   <211> 40
   <212> DNA
   <213> Artificial sequence
<220>
   <223> primer
<400> 49
   gatgcaaggt cgcatatgag ctgttttggg cgtcgatcca 40
<210> 50 .
   <211> 51
   <212> DNA
   <213> Artificial sequence
<220>
   <223> primer
<400> 50
   aattctaata cgactcacta tagggagaag gcagttggcg gggacggcta a 51
<210> 51
   <211> 40
   <212> DNA
   <213> Artificial sequence
<220>
   <223> primer
<400> 51
   gatgcaaggt cgcatatgag ggcgtcgatc cactgcggga 40
<210> 52
   <211> 51
   <212> DNA
   <213> Artificial sequence
<220>
   <223> primer
<400> 52
   aattctaata cgactcacta tagggagaag gggtgggcaa gaagaagtcg a 51
<210> 53
   <211> 40
   <212> DNA
   <213> Artificial sequence
<220>
   <223> primer
<400> 53
   gatgcaaggt cgcatatgag ctccagcacc tccttggtca 40
<210> 54
   <211> 51
   <212> DNA
   <213> Artificial sequence
<220>
   <223> primer
<400> 54
   aattctaata cgactcacta tagg,gagaag gccaggctga taccacgtct a 51
<210> 55
   <211> 40
   <212> DNA
   <213> Artificial sequence
<220>
   <223> primer
<400> 55
   gatgcaaggt cgcatatgag cctccttggt caattcgtta 40
<210> 56
   <211> 51
   <212> DNA
   <213> Artificial sequence
<220>
   <223> primer
<400> 56
   aattctaata cgactcacta tagggagaag gaagtcaacc ctgctccatg a 51
<210> 57
   <211> 1321
   <212> DNA
   <213> Mycobacterium tuberculosis
<220>
   <221> CDS
   <222> (542) .. (1165)
   <223>
<400> 57
   gcttagttgg tgagattgcg aaagccgagg gtcgatcccc ggaggtgctc gacgcggccg    60
   ctgatcgctt cggtcggcgg gttgaccgtg gtcactgttt tgggcgtcga tccactgcgg   120
   gaattcccac taccacgtcc ggccggatca ccggcgactc gcggtgcacg gcccgctcca   180
   gcacctcctt ggtcaattcg ttagccgtcc ccgccaactg cccagccgtc gacttcttct   240
   tgcccaccca ccccatagac cttcgccaca cagcgccttc cgtccaccca acagcggtcc   300
   gatgacggac ccccgacggg gacttcagcg accaggaacg cgcccataga cgtggtatca   360
   gcctgggggc gtcctggtag cctatgccgt ccgccctggg gcatcgaccc caaggtcgtt   420
   gttgcgacgc gagcggtcat ggagcagggt tgacttgtca agctagagcc agcccatcgc   480
   gtgggaggca cccgcgcgaa aagaaacatc ggacgatcat ttcatcgaag gaaggaatgc   540
   c gtg gcc gaa tac acc ttg cca gac ctg gac tgg gac tac gga gca ctg   589
     Val Ala Glu Tyr Thr Leu Pro Asp Leu Asp Trp Asp Tyr Gly Ala Leu
     1               5                   10                  15
   gaa ccg cac atc tcg ggt cag atc aac gag ctt cac cac agc aag cac     637
   Glu Pro His Ile Ser Gly Gln Ile Asn Glu Leu His His Ser Lys His
               20                  25                  30
   cac gcc acc tac gta aag ggc gcc aat gac gcc gtc gcc aaa ctc gaa     685
   His Ala Thr Tyr Val Lys Gly Ala Asn Asp Ala Val Ala Lys Leu Glu
           35                  40                  45
   gag gcg cgc gcc aag gaa gat cac tca gcg atc ttg ctg aac gaa aag     733
   Glu Ala Arg Ala Lys Glu Asp His Ser Ala Ile Leu Leu Asn Glu Lys
       50                  55                  60
   aat cta gct ttc aac ctc gcc ggc cac gtc aat cac acc atc tgg tgg     781
   Asn Leu Ala Phe Asn Leu Ala Gly His Val Asn His Thr Ile Trp Trp
   65                  70                  75                  80
   aag aac ctg tcg cct aac ggt ggt gac aag ccc acc ggc gaa ctc gcc     829
   Lys Asn Leu Ser Pro Asn Gly Gly Asp Lys Pro Thr Gly Glu Leu Ala
                   85                  90                  95
   gca gcc atc gcc gac gcg ttc ggt tcg ttc gac aag ttc cgt gcg cag     877
   Ala Ala Ile Ala Asp Ala Phe Gly Ser Phe Asp Lys Phe Arg Ala Gln
               100                 105                 110
   ttc cac gcg gcc gct acc acc gtg cag ggg tcg ggc tgg gcg gca ctg      925
   Phe His Ala Ala Ala Thr Thr Val Gln Gly Ser Gly Trp Ala Ala Leu
           115                     120                     125
   ggc tgg gac aca ctc ggc aac aag ctg ctg ata ttc cag gtt tac gac      973
   Gly Trp Asp Thr Leu Gly Asn Lys Leu Leu Ile Phe Gln Val Tyr Asp
       130                     135                     140
   cac cag acg aac ttc ccg cta ggc att gtt ccg ctg ctg ctg ctc gac     1021
   His Gln Thr Asn Phe Pro Leu Gly Ile Val Pro Leu Leu Leu Leu Asp
   145                 150                     155                 160
   atg tgg gaa cac gcc ttc tac ctg cag tac aag aac gtc aaa gtc gac     1069
   Met Trp Glu His Ala Phe Tyr Leu Gln Tyr Lys Asn Val Lys Val Asp
                   165                     170                     175
   ttt gcc aag gcg ttt tgg aac gtc gtg aac tgg gcc gat gtg cag tca     1117
   Phe Ala Lys Ala Phe Trp Asn Val Val Asn Trp Ala Asp Val Gln Ser
               180                     185                     190
   cgg tat gcg gcc gcg acc tcg cag acc aag ggg ttg ata ttc ggc tga     1165
   Arg Tyr Ala Ala Ala Thr Ser Gln Thr Lys Gly Leu Ile Phe Gly
           195                     200                     205
   ccccgctgcc gcaagcgtcg ggctcagtat tccggagtcg cgcatcacca tcgcccttat   1225
   cctggcctta tattgcagct ttgtgaacac ggccgcggtg gccgtgtcga gttgcagggc   1285
   gcgtaaacca cgcgcatgct tggttactcg agctac                             1321
<210> 58
   <211> 207
   <212> PRT
   <213> Mycobacterium tuberculosis
<400> 58
   Val Ala Glu Tyr Thr Leu Pro Asp Leu Asp Trp Asp Tyr Gly Ala Leu
   1               5                   10                  15
   Glu Pro His Ile Ser Gly Gln Ile Asn Glu Leu His His Ser Lys His
               20                  25                  30
   His Ala Thr Tyr Val Lys Gly Ala Asn Asp Ala Val Ala Lys Leu Glu
           35                  40                  45
   Glu Ala Arg Ala Lys Glu Asp His Ser Ala Ile Leu Leu Asn Glu Lys
       50                  55                  60
   Asn Leu Ala Phe Asn Leu Ala Gly His Val Asn His Thr Ile Trp Trp
   65                  70                  75                  80
   Lys Asn Leu Ser Pro Asn Gly Gly Asp Lys Pro Thr Gly Glu Leu Ala
                   85                  90                  95
   Ala Ala Ile Ala Asp Ala Phe Gly Ser Phe Asp Lys Phe Arg Ala Gln
               100                 105                 110
   Phe His Ala Ala Ala Thr Thr Val Gln Gly Ser Gly Trp Ala Ala Leu
           115                 120                 125
   Gly Trp Asp Thr Leu Gly Asn Lys Leu Leu Ile Phe Gln Val Tyr Asp
       130                 135                 140
   His Gln Thr Asn Phe Pro Leu Gly Ile Val Pro Leu Leu Leu Leu Asp
   145                 150                 155                 160
   Met Trp Glu His Ala Phe Tyr Leu Gln Tyr Lys Asn Val Lys Val Asp
                   165                 170                 175
   Phe Ala Lys Ala Phe Trp Asn Val Val Asn Trp Ala Asp Val Gln Ser
               180                 185                 190
   Arg Tyr Ala Ala Ala Thr Ser Gln Thr Lys Gly Leu Ile Phe Gly
           195                 200                 205
<210> 59
   <211> 900
   <212> DNA
   <213> Mycobacterium avium ssp. paratuberculosis
<220>
   <221> CDS
   <222> (260)..(883)
   <223>
<400> 59
   tcgcccgcgt ggaaccacag ctacagcgtc gcggcactgc gcgccggatt gatcgcactg      60
   gcgctgctgg cggtattggc cctcatcgtc ttgtcttgaa gtcctgtctt gaaacaccgc     120
   tgagcgggta tccgccttcc ggctcagcgg ggtcgttctt gcggtgcgcg cggtcatgga     180
   gcagggttga taacgcccgg ccgccgcgag gtggcccgcg cgactgagcg tcaagacgac     240
   aatcgaagaa aggaatgcc gtg gct gaa tac acc ctg ccc gac ctg gac tgg      292
                        Val Ala Glu Tyr Thr Leu Pro Asp Leu Asp Trp
                        1               5                   10
   gac tat gca gcg ttg gaa ccg cac atc tcg ggg cag atc aac gag atc        340
   Asp Tyr Ala Ala Leu Glu Pro His Ile Ser Gly Gln Ile Asn Glu Ile
               15                  20                  25
   cac cac acc aag cac cac gcc acc tac gtc aaa ggc gtg aac gac gct        388
   His His Thr Lys His His Ala Thr Tyr Val Lys Gly Val Asn Asp Ala
           30                  35                  40
   ctt gcc aag ctc gaa gag gcc cgc gcc aac gag gac cac gct gcg atc        436
   Leu Ala Lys Leu Glu Glu Ala Arg Ala Asn Glu Asp His Ala Ala Ile
       45                  50                  55
   ttc ctg aac gaa aag aac ctc gcc ttc cac ctg ggc ggc cac gtc aac        484
   Phe Leu Asn Glu Lys Asn Leu Ala Phe His Leu Gly Gly His Val Asn
   60                  65                  70                  75
   cac tcg atc tgg tgg aag aac ctg tcg ccg gac ggc ggt gac aag ccc        532
   His Ser Ile Trp Trp Lys Asn Leu Ser Pro Asp Gly Gly Asp Lys Pro
                   80                  85                  90
   acc ggc gag ctg gcc gcc gcg atc gac gac gcg ttc ggc tcc ttc gac        580
   Thr Gly Glu Leu Ala Ala Ala Ile Asp Asp Ala Phe Gly Ser Phe Asp
               95                  100                 105
   aag ttc cgg gcg caa ttc agc gcc gcc gcc aac ggc ctg cag ggc tcc        628
   Lys Phe Arg Ala Gln Phe Ser Ala Ala Ala Asn Gly Leu Gln Gly Ser
           110                 115                 120
   ggc tgg gcg gtg ctg ggt tat gac acc gtg ggc agc cgg ttg ctg acc        676
   Gly Trp Ala Val Leu Gly Tyr Asp Thr Val Gly Ser Arg Leu Leu Thr
       125                 130                 135
   ttc cag ctc tac gac cag cag gcc aac gtc ccg ctg ggc atc atc ccg        724
   Phe Gln Leu Tyr Asp Gln Gln Ala Asn Val Pro Leu Gly Ile Ile Pro
   140                 145                 150                 155
   ctg ctg cag gtc gac atg tgg gag cac gcg ttc tac ctg cag tac aag        772
   Leu Leu Gln Val Asp Met Trp Glu His Ala Phe Tyr Leu Gln Tyr Lys
                   160                 165                 170
   aac gtc aag gcg gat tac gtc aag gcg ttc tgg aac gtg gtc aac tgg        820
   Asn Val Lys Ala Asp Tyr Val Lys Ala Phe Trp Asn Val Val Asn Trp
               175                 180                 185
   gcg gac gtg cag aag cgg tac gcc gcc gcc act tcc aag gcc caa ggc        868
   Ala Asp Val Gln Lys Arg Tyr Ala Ala Ala Thr Ser Lys Ala Gln Gly
           190                 195                 200
   ctg atc ttc ggc tga ctttcctctc gatcggc                                 900
   Leu Ile Phe Gly
       205
<210> 60
   <211> 207
   <212> PRT
   <213> Mycobacterium avium ssp. paratuberculosis
<400> 60
   Val Ala Glu Tyr Thr Leu Pro Asp Leu Asp Trp Asp Tyr Ala Ala Leu
   1               5                   10                  15
   Glu Pro His Ile Ser Gly Gln Ile Asn Glu Ile His His Thr Lys His
               20                  25                  30
   His Ala Thr Tyr Val Lys Gly Val Asn Asp Ala Leu Ala Lys Leu Glu
           35                  40                  45
   Glu Ala Arg Ala Asn Glu Asp His Ala Ala Ile Phe Leu Asn Glu Lys
       50                  55                  60
   Asn Leu Ala Phe His Leu Gly Gly His Val Asn His Ser Ile Trp Trp
   65                  70                  75                  80
   Lys Asn Leu Ser Pro Asp Gly Gly Asp Lys Pro Thr Gly Glu Leu Ala
                   85                  90                  95
   Ala Ala Ile Asp Asp Ala Phe Gly Ser Phe Asp Lys Phe Arg Ala Gln
               100                 105                 110
   Phe Ser Ala Ala Ala Asn Gly Leu Gln Gly Ser Gly Trp Ala Val Leu
           115                 120                 125
   Gly Tyr Asp Thr Val Gly Ser Arg Leu Leu Thr Phe Gln Leu Tyr Asp
       130                 135                 140
   Gln Gln Ala Asn Val Pro Leu Gly Ile Ile Pro Leu Leu Gln Val Asp
   145                 150                 155                 160
   Met Trp Glu His Ala Phe Tyr Leu Gln Tyr Lys Asn Val Lys Ala Asp
                   165                 170                 175
   Tyr Val Lys Ala Phe Trp Asn Val Val Asn Trp Ala Asp Val Gln Lys
               180                 185                 190
   Arg Tyr Ala Ala Ala Thr Ser Lys Ala Gln Gly Leu Ile Phe Gly
           195                 200                 205
<210> 61
   <211> 20
   <212> DNA
   <213> artificial sequence
<220>
   <223> primer
<400> 61
   gccgaataca ccttgccaga 20
<210> 62
   <211> 20
   <212> DNA
   <213> artificial sequence
<220>
   <223> primer
<400> 62
   cgtggtgctt gctgtggtga 20
<210> 63
   <211> 20
   <212> DNA
   <213> artificial sequence
<220>
   <223> probe
<400> 63
   caattcagcg ccgccgccaa 20
<210> 64
   <211> 20
   <212> DNA
   <213> artificial sequence
<220>
   <223> primer
<400> 64
   acctggactg ggactacgga 20
<210> 65
   <211> 20
   <212> DNA
   <213> artificial sequence
<220>
   <223> primer
<400> 65
   tcattggcgc cctttacgta . 20
<210> 66
   <211> 20
   <212> DNA
   <213> artificial sequence
<220>
   <223> probe
<400> 66
   aattcagcgc cgccgccaac 20
<210> 67
   <211> 20
   <212> DNA
   <213> artificial sequence
<220>
   <223> primer
<400> 67
   tggactggga ctacggagca 20
<210> 68
   <211> 20
   <212> DNA
   <213> artificial sequence
<220>
   <223> primer
<400> 68
   cgagtttggc gacggcgtca 20
<210> 69
   <211> 20
   <212> DNA
   <213> artificial sequence
<220>
   <223> primer
<400> 69
   aagttccggg cgcaattcag 20
<210> 70
   <211> 40
   <212> DNA
   <213> artificial sequence
<220>
   <223> primer
<400> 70
   gatgcaaggt cgcatatgag gccgaataca ccttgccaga 40
<210> 71
   <211> 51
   <212> DNA
   <213> artificial sequence
<220>
   <223> primer
<400> 71
   aattctaata cgactcacta tagggagaag gcgtggtgct tgctgtggtg a 51
<210> 72
   <211> 40
   <212> DNA
   <213> artificial sequence
<220>
   <223> primer
<400> 72
   gatgcaaggt cgcatatgag tggactggga ctacggagca 40
<210> 73
   <211> 51 .
   <212> DNA
   <213> artificial sequence
<220>
   <223> primer
<400> 73
   aattctaata cgactcacta tagggagaag gcgagtttgg cgacggcgtc a 51
<210> 74
   <211> 20
   <212> DNA
   <213> artificial sequence
<220>
   <223> primer
<400> 74
   gcgccaagga agatcactca 20
<210> 75
   <211> 20
   <212> DNA
   <213> artificial sequence
<220>
   <223> primer
<400> 75
   ggcgacaggt tcttccacca 20
<210> 76
   <211> 20
   <212> DNA
   <213> artificial sequence
<220>
   <223> probe
<400> 76
   gaatctagct ttcaacctcg 20
<210> 77
   <211> 20
   <212> DNA
   <213> artificial sequence
<220>
   <223> primer
<400> 77
   caaggaagat cactcagcga 20
<210> 78
   <211> 20
   <212> DNA
   <213> artificial sequence
<220>
   <223> primer
<400> 78
   gtcaccaccg ttaggcgaca 20
<210> 79
   <211> 20
   <212> DNA
   <213> artificial sequence
<220>
   <223> probe
<400> 79
   atctagcttt caacctcgcc 20
<210> 80
   <221> 20
   <212> DNA
   <213> artificial sequence
<220>
   <223> primer
<400> 80
   agcgatcttg ctgaacgaaa 20
<210> 81
   <211> 20
   <212> DNA
   <213> artificial sequence
<220>
   <223> primer
<400> 81
   tgggcttgtc accaccgtta 20
<210> 82
   <211> 20
   <212> DNA
   <213> artificial sequence
<220>
   <223> probe
<400> 82
   ggccacgtca atcacaccat 20
<210> 83
   <211> 20
   <212> DNA
   <213> artificial sequence
<220>
   <223> primer
<400> 83
   ttccagctct acgaccagca 20
<210> 84
   <211> 20
   <212> DNA
   <213> artificial sequence
<220>
   <223> primer
<400> 84
   ttggaagtgg cggcggcgta 20
<210> 85
   <211> 20
   <212> DNA
   <213> artificial sequence
<220>
   <223> probe
<400> 85 20
   aaggcggatt acgtcaaggc 20
<210> 86
   <211> 20
   <212> DNA
   <213> artificial sequence
<220>
   <223> primer
<400> 86 20
   ctctacgacc agcaggccaa 20
<210> 87
   <211> 20
   <212> DNA
   <213> artificial sequence
<220>
   <223> primer
<400> 87 20
   ctgcacgtcc gcccagttga 20
<210> 88
   <211> 20
   <212> DNA
   <213> artificial sequence
<220>
   <223> probe
<400> 88 20
   aagaacgtca aggcggatta 20
<210> 89
   <211> 20
   <212> DNA
   <213> artificial sequence
<220>
   <223> primer
<400> 89
   tacgaccagc aggccaacgt 20
<210> 90
   <211> 20
   <212> DNA
   <213> artificial sequence
<220>
   <223> probe
<400> 90
   cacgtccgcc cagttgacca 20
<210> 91
   <211> 20
   <212> DNA
   <213> artificial sequence
<220>
   <223> probe
<400> 91
   gtacaagaac gtcaaggcgg 20
<210> 92
   <211> 20
   <212> DNA
   <213> artificial sequence
<220>
   <223> primer
<400> 92
   tcgagctgcc agtgtggtca 20
<210> 93
   <211> 20
   <212> DNA
   <213> artificial sequence
<220>
   <223> primer
<400> 93
   acgagaatgg aagggggaca 20
<210> 94
   <211> 20
   <212> DNA
   <213> artificial sequence
<220>
   <223> probe
<400> 94
   aacagggtcg gatcggccta 20
<210> 95
   <211> 20
   <212> DNA
   <213> artificial sequence
<220>
   <223> primer
<400> 95
   ccagtgtggt catttccata 20
<210> 96
   <211> 20
   <212> DNA
   <213> artificial sequence
<220>
   <223> primer
<400> 96
   agggcaccac gagaatggaa 20
<210> 97
   <211> 20
   <212> DNA
   <213> artificial sequence
<220>
   <223> probe
<400> 97
   gcgcggaggt cgaaatggaa 20
<210> 98
   <211> 561
   <212> DNA
   <213> Mycobacterium tuberculosis
<220>
   <221> CDS
   <222> (1)..(561)
   <223>
<400> 98
   atg cgg gcg ttg atc atc gtc gac gtg cag aac gac ttc tgc gag ggt      48
   Met Arg Ala Leu Ile Ile Val Asp Val Gln Asn Asp Phe Cys Glu Gly
   1               5                   10                  15
   ggc tcg ctg gcg gta acc ggt ggc gcc gcg ctg gcc cgc gcc atc agc      96
   Gly Ser Leu Ala Val Thr Gly Gly Ala Ala Leu Ala Arg Ala Ile Ser
               20                  25                  30
   gac tac ctg gcc gaa gcg gcg gac tac cat cac gtc gtg gca acc aag      144
   Asp Tyr Leu Ala Glu Ala Ala Asp Tyr His His Val Val Ala Thr Lys
           35                  40                  45
   gac ttc cac atc gac ccg ggt gac cac ttc tcc ggc aca ccg gac tat      192
   Asp Phe His Ile Asp Pro Gly Asp His Phe Ser Gly Thr Pro Asp Tyr
       50                  55                  60
   tcc tcg tcg tgg cca ccg cat tgc gtc agc ggt act ccc ggc gcg gac      240
   Ser Ser Ser Trp Pro Pro His Cys Val Ser Gly Thr Pro Gly Ala Asp
   65                  70                  75                  80
   ttc cat ccc agt ctg gac acg tcg gca atc gag gcg gtg ttc tac aag      288
   Phe His Pro Ser Leu Asp Thr Ser Ala Ile Glu Ala Val Phe Tyr Lys
                   85                  90                  95
   ggt gcc tac acc gga gcg tac agc ggc ttc gaa gga gtc gac gag aac      336
   Gly Ala Tyr Thr Gly Ala Tyr Ser Gly Phe Glu Gly Val Asp Glu Asn
               100                 105                 110
   ggc acg cca ctg ctg aat tgg ctg cgg caa cgc ggc gtc gat gag gtc      384
   Gly Thr Pro Leu Leu Asn Trp Leu Arg Gln Arg Gly Val Asp Glu Val
           115                 120                 125
   gat gtg gtc ggt att gcc acc gat cat tgt gtg cgc cag acg gcc gag      432
   Asp Val Val Gly Ile Ala Thr Asp His Cys Val Arg Gln Thr Ala Glu
       130                 135                 140
   gac gcg gta cgc aat ggc ttg gcc acc agg gtg ctg gtg gac ctg aca      480
   Asp Ala Val Arg Asn Gly Leu Ala Thr Arg Val Leu Val Asp Leu Thr
   145                 150                 155                 160
   gcg ggt gtg tcg gcc gat acc acc gtc gcc gcg ctg gag gag atg cgc      528
   Ala Gly Val Ser Ala Asp Thr Thr Val Ala Ala Leu Glu Glu Met Arg
                   165                 170                 175
   acc gcc agc gtc gag ttg gtt tgc agc tcc tga                          561
   Thr Ala Ser Val Glu Leu Val Cys Ser Ser
               180                 185
<210> 99
   <211> 186
   <212> PRT
   <213> Mycobacterium tuberculosis
<400> 99

## Description

The present invention is concerned with methods for detecting the presence of microorganisms, particularly slow-growing bacteria of the genus *Mycobacterium,* by induction of mRNA expression and detection of the induced mRNA.

### BACKGROUND TO THE INVENTION

Despite effective antimicrobial therapy, tuberculosis remains a major source of morbidity and mortality throughout the world and is increasing worldwide. Early diagnosis and prompt initiation of treatment for tuberculosis meningitis improve the outcome of the disease, but the detection of *Mycobacterium tuberculosis* in cerebrospinal fluid (CSF) by conventional methods remains a challenge. The challenge for diagnosis of mycobacterium is that the low number of *M*. *tuberculosis* organisms and their slow growth limit detection by acid-fast stains and culture techniques (Molavi A and J.L. LeFrock., Med. Clin. North. Am. 69:315-331. 1985).

Several research groups have described PCR assays for the sensitive and specific detection of *Mycobacterium tuberculosis* in clinical samples. Direct identification of mycobacteria in clinical samples by PCR amplification of a specific target nucleic acid sequence offers the potential for same-day diagnosis of infection. PCR techniques have been applied on real clinical samples but are limited by the small sample size that can be tested, the low number of organisms present in clinical samples, false-positive results due to contamination, and the presence of PCR inhibitors (Kox et al., J.Clin Micro 32:672-678. 1994; US 5525463.

The predictive value for detection is greatly dependent on the incidence of *M. tuberculosis*-positive samples among the clinical specimens investigated. Grosset and Mouton recommended that PCR should not be used for the routine diagnosis of tuberculosis until the technique is robust and internal and external quality control procedures exists (Grosset J. and Y Mouton., Tubercle Lung dis 76:183-184. 1995). In an international collaborative quality control study among 30 laboratories only five laboratories correctly identified the presence or absence of mycobacterial DNA in all 20 samples (Noordhoek G.T. J. Clin. Microbiology 34:2522-2525. 1996). This study also showed that lack of specificity was more of a problem than lack of sensitivity. As yet, amplification-based detection techniques have not replaced the conventional diagnostic techniques (Piersimoni et al., 36:3601-3604. 1998. J. Clin Microbiology; see also Piersimoni et al. 35:193-196. 1997. J. Clin Microbiology).

*Mycobacterium paratuberculosis* is a chronic enteric pathogen that can affect many different species of animals including primates (Chiodini et al., Cornell Vet. 74:218-262. 1984). The organism was first identified 100 years ago as the cause of chronic inflammation of the intestine in a German cow. Classification of Johne's disease or paratuberculosis in animals is characterized by the presence in the affected intestine of millions of bacillary-form acid-fast mycobacteria with macrophages but with little additional inflammatory cell infiltrate. M. *paratuberculosis* is rarely cultured in its bacillary form from these animals. This clinicopathological spectrum of pluribacillary-paucimicrobial paratuberculosis in animals is reminiscent of the extremes represented by the lepromatous and tuberculoid forms of leprosy in humans. Progress in our understanding of *M*. *tuberculosis* and of the diseases it causes has been considerably retarded over the years by the sometimes-great difficulty of identifying this agent by conventional culture in the laboratory (Chiodini et al., Clin. Microbiol. Rev. 2:90-117. 1989). There may be a high risk, particularly in peak time, that residual *M*. *tuberculosis* will be present in retail pasteurized cows milk in the UK.

Thus, there remains a need for sensitive, specific methods of detecting *Microbacterium* spp., particularly *M*. *tuberculosis*.

### DESCRIPTION OF THE INVENTION

In accordance with a first aspect of the invention there is provided a method for detecting the presence of a microorganism in a test sample which comprises exposing the test sample to an inducer which is capable of inducing expression of at least one gene in the microorganism and testing for the presence of mRNA transcribed from a gene which is induced by exposure to the said inducer.

In a preferred embodiment the microorganism may be a slow-growing bacterium. Preferred slow-growing bacteria include, for example, *Borreliae, Pediococcus, Mycoplasma*, and strains of *Mycobacteria*.

The method is most preferred for the detection of *Mycobacteria Spp.,* particularly *Mycobacterium tuberculosis, Mycobacterium paratuberculosis* (e.g. *M. avium subsp. paratuberculosis*) and *Mycobacterium bovis*.

The "test sample" suspected of containing the microorganism will most commonly be a clinical sample taken from an individual to be tested for the presence of the microorganism.

Testing for the presence of specific mRNA sequences will preferably be carried out on a preparation of nucleic acid isolated from the test sample. This preparation of nucleic acid must include mRNA, however it need not be a preparation of purified poly A+ mRNA and preparations of total RNA or even preparations of total nucleic acid containing both RNA and genomic DNA are also suitable as starting material, depending on the nature of the technique used to test for the presence of the specific mRNA. Essentially any technique known in the art for the isolation of a preparation of nucleic acid including mRNA may be used to isolate nucleic acid from the test sample. A preferred technique is the "Boom" isolation method described in US-A-5,234,809 and EP-B-0389,063. This method, which can be used to isolate a nucleic acid preparation containing both RNA and DNA, is based on the nucleic acid binding properties of silicon dioxide particles in the presence of the chaotropic agent guanidine thiocyanate (GuSCN).

In a preferred embodiment the step of testing for the presence of mRNA transcribed from a gene which is induced by exposure to the inducer comprises performing an amplification reaction to amplify the whole or a portion of the mRNA and detecting specific products of the amplification reaction. Most preferably, the amplification reaction is reverse transcription-polymerase chain reaction (RT-PCR) or nucleic acid sequence-based amplification (NASBA).

RT-PCR is well known in the art for the detection of specific mRNA sequences. Protocols for performing RT-PCR and detecting the specific products of RT-PCR reactions may be found in standard texts such as, for example, PCR Protocols: A Guide to Methods and Applications, Eds Innis et al., Academic Press, San Diego, CA, 1990; PCR in Bioanalysis: Methods in Molecular Biology, Vol. 92, S.J. Smeltzer, Ed., Humana Press, Totowa, NJ., 1998. NASBA is a relatively new method for the amplification of RNA (see Compton, Nature. 350: 91-92 (1991)). NASBA is well known by persons of ordinary skill in the art and is described, for example, in US-A-5,409,818. NASBA is an effective procedure for generating large quantities of a target RNA sequence *in vitro,* allowing detection of target RNA sequences that are present in very low concentrations in the original test sample.

The NASBA method is based on the use of similar primer-sets to those used for PCR but one primer is modified with a promoter sequence, for example a T7 promoter. The sensitivity and specificity of the NASBA amplification has been shown to be the same as for PCR and better then most RT-PCR protocols. Since the NASBA method is an isothermal assay and it is dependent on RNase H it cannot amplify DNA.

NASBA is preferably carried out according to the following procedure:
(a) assemble a reaction mixture comprising a NASBA P1 and NASBA P2 primer pair suitable for use in amplification of a region of the target microorganism mRNA by NASBA (see below), an RNA directed DNA polymerase, a ribonuclease that hydrolyses the RNA strand of an RNA-DNA hybrid without hydrolysing single or double stranded RNA or DNA, an RNA polymerase that recognises a promoter sequence present in the NASBA P1 primer and ribonucleoside and deoxyribonucleoside triphosphates;
(b) incubate the reaction medium with a preparation of nucleic acid isolated from a test sample suspected of containing the microorganism under reaction conditions which permit a NASBA amplification reaction; and
(c) detect and/or quantitatively measure any microorganism-specific product of the NASBA amplification reaction.

Detection of the specific product(s) of a NASBA reaction (i.e. sense and/or antisense copies of the target RNA) may be carried out in a number of different ways. In one approach the NASBA product(s) may be detected with the use of a target-specific hybridisation probe capable of specifically annealing to the NASBA product. The hybridisation probe may be attached to a revealing label, for example a fluorescent, luminescent, radioactive or chemiluminescent compound or an enzyme label or any other type of label known to those of ordinary skill in the art. The precise nature of the label is not critical, but it should be capable of producing a signal detectable by external means, either by itself or in conjunction with one or more additional substances (e.g. the substrate for an enzyme).

Also within the scope of the invention is so-called "real-time NASBA" which allows continuous monitoring of the formation of the product of the NASBA reaction over the course of the reaction. This may be achieved using a "molecular beacons" probe comprising a target-specific sequence capable of annealing to the NASBA product, a hairpin forming oligonucleotide sequence and a pair of fluorescer/quencher moieties, as described in WO 95/13399. Molecular beacons technology is described more fully below. If the molecular beacons probe is added to the reaction mixture prior to amplification it may be possible to monitor the formation of the NASBA product in real-time (see Leone et al., Nucleic Acids Research, 1998, Vol 26, 2150-2155).

In a further approach, the molecular beacons technology may be incorporated into one of the oligonucleotide primers used in the NASBA amplification, as described below, in order to facilitate real-time monitoring of the NASBA reaction without the need for a separate hybridisation probe.

In a still further approach the products of the NASBA reaction may be monitored using a generic labelled detection probe which hybridises to a nucleotide sequence in the 5' terminus of primer 2. This is equivalent to the "NucliSens™" detection system supplied by Organon Teknika. In this system specificity for NASBA products derived from the target mRNA may be conferred by using target-specific capture probes comprising probe oligonucleotides as described herein attached to a solid support such as a magnetic microbead. Most preferably the generic labelled detection probe is the ECL™ detection probe supplied by Organon Teknika. NASBA amplicons are hybridized to the HPV-specific capture probes and the generic ECL probe (via a complementary sequence on primer 2). Following hybridization the bead/amplicon/ECL probe complexes may be captured at the magnet electrode of an automatic ECL reader (e.g. the NucliSens™ reader supplied by Organon Teknika. Subsequently, a voltage pulse triggers the ECL™ reaction.

The NASBA reaction has already been used successfully within the prior art for the amplification and detection of mycobacteria (see van der Vliet et al., Journal of General Microbiology, 1993, Vol 139, 2423-2429), and has not been reported in combination with inducing agents.

The "induced gene or gene(s)" may be any gene(s) which can be induced by an external stimulus such as, for example, a chemical agent or environmental change.

Induction of expression of one or more genes, followed by specific detection of transcripts of at least one induced gene, provides an additional level of amplification and thus increased sensitivity as compared to methods which rely on detection of mRNA expression without an induction step.

The "inducer" may be essentially anything which is capable of inducing expression of one or more genes in the target species of microorganism. The inducer may be a chemical substance or an environmental stimulus such as, for example, exposure to light of a particular wavelength.

The test sample to be tested for the presence of the microorganism is exposed to the inducer for a period of time sufficient to produce the desired induction of gene expression. The precise exposure time required will vary depending on the nature of the inducer and the target gene to be induced. For any given inducer/gene combination a suitable exposure time may be determined empirically by routine experiment. Following exposure to the inducer, nucleic acid may be isolated from the test sample as described above.

In one embodiment, the invention provides a method of detecting microorganisms which is based on induction of expression of a gene encoding the enzyme superoxide dismutase by exposure of the test sample to oxygen or hydrogen peroxide, followed by testing for the presence of mRNA transcripts corresponding to this gene. This method is preferred for the detection of *Mycobacteria*, most advantageously *M. tuberculosis* or *M. paratuberculosis,* but may be adapted for the detection of any microorganism having a superoxide dismutase gene, wherein expression of this gene is induced by exposure to oxygen or hydrogen peroxide. In *M. tuberculosis* the gene encoding superoxide dismutase is denoted sodA (GenBank accession AF061030; SEQ ID NO:57 and 58). In *M. avium subsp. paratuberculosis* the gene encoding superoxide dismutase is denoted sod (GenBank accession AF180816; SEQ ID NO:59 and 60). SodA/sod homologs in other species of microorganism may be identified by using methods well known to those skilled in the art, for example by searching publicly accessible sequence databases and/or by screening the genomic DNA directly.

The step of testing for the presence of mRNA transcripts corresponding to the superoxide dismutase gene may be accomplished by amplifying the whole or a portion of the mRNA by RT-PCR or NASBA and detecting superoxide dismutase-specific amplification products.

Suitable primer-sets for use in the amplification of sodA mRNA by RT-PCR or NASBA are given below. These primer sets may be used in the detection of all *Mycobaterium spp*. but are particularly suitable for use in the detection of *M. tuberculosis* and *M. bovis.*
Primer sets for detection by NASBA:
a NASBA P1 primer comprising SEQ ID NO:20 and a NASBA P2 primer comprising SEQ ID NO:19;
a NASBA P1 primer comprising SEQ ID NO:26 and a NASBA P2 primer comprising SEQ ID NO:25;
a NASBA P1 primer comprising SEQ ID NO:32 and a NASBA P2 primer comprising SEQ ID NO:31;
a NASBA P1 primer comprising SEQ ID NO:38 and a NASBA P2 primer comprising SEQ ID NO:37;
a NASBA P1 primer comprising SEQ ID NO:61 and a NASBA P2 primer comprising SEQ ID NO:62;
a NASBA P1 primer comprising SEQ ID NO:64 and a NASBA P2 primer comprising SEQ ID NO:65;
NASBA P1 primer 4-86 and a NASBA P2 primer comprising SEQ ID NO:61, preferably primer 4-85;
primer 4-92 and a NASBA P2 primer comprising SEQ ID NO:64, preferably primer 4-91;
primer 4-104 and a NASBA P2 primer comprising SEQ ID NO:19, preferably primer 4-103;
primer 4-122 and a NASBA P2 primer comprising SEQ ID NO:31, preferably primer 4-115;
Primer sets for detection by RT-PCR:
a PCR primer comprising SEQ ID NO:20 and PCR primer comprising SEQ ID NO:19;
a PCR primer comprising SEQ ID NO:26 and a PCR primer comprising SEQ ID NO:25;
a PCR primer comprising SEQ ID NO:32 and a PCR primer comprising SEQ ID NO:31;
a PCR primer comprising SEQ ID NO:38 and a PCR primer comprising SEQ ID NO:37;
a PCR primer comprising SEQ ID NO:61 and a PCR primer comprising SEQ ID NO:62; or
a PCR primer comprising SEQ ID NO:64 and a PCR primer comprising SEQ ID NO:65.

In each case the "SEQ ID NOs:" refer to the sequences set forth in Table 1 and the "primer numbers" to the primers listed in Table 2.

Probe oligonucleotides suitable for use in the detection of the products of NASBA and RT-PCR reactions performed using these specific primer sets are also provided by the invention, as discussed below.

In a further embodiment, the invention provides a method of detecting microorganisms which is based on induction of expression of the pncA gene by exposure of the test sample to a chemical inducer which may be isopropyl-B-D-thiogalactopyranoside or pyrazinamide, followed by testing for the presence of mRNA transcripts corresponding to the pncA gene. This method is again preferred for the detection of *Mycobacteria Spp.* The complete cDNA sequence for *M*. *tuberculosis* pncA is available under GenBank accession number U59967 (see also SEQ ID NOs:98 and 99). PncA homologs in other species of microorganism may be identified by using methods well known to those skilled in the art.

Again, the step of testing for the presence of mRNA transcripts corresponding to the pncA gene may be accomplished by amplifying the whole or a portion of the mRNA by RT-PCR or NASBA and detecting pncA-specific amplification products.

Suitable primer-sets specifically for use in the amplification of pncA mRNA from *Mycobacterium tuberculosis* by RT-PCR or NASBA are given below.
Primer sets for detection by NASBA:
a NASBA P1 primer comprising SEQ ID NO:2 and a NASBA P2 primer comprising SEQ ID NO:1;
a NASBA P1 primer comprising SEQ ID NO:8 and a NASBA P2 primer comprising SEQ ID NO:7;
a NASBA P1 primer comprising SEQ ID NO:14 and a NASBA P2 primer comprising SEQ ID NO:13;
primer 4-2 and a NASBA P2 primer comprising SEQ ID NO:1, preferably primer 4-1;
primer 4-8 and a NASBA P2 primer comprising SEQ ID NO:7, preferably primer 4-7; or
primer 4-14 and a NASBA P2 primer comprising SEQ ID NO:13, preferably primer 4-15.
Primer sets for detection by RT-PCR:
a PCR primer comprising SEQ ID NO:2 and a PCR primer comprising SEQ ID NO:1;
a PCR primer comprising SEQ ID NO:8 and a PCR primer comprising SEQ ID NO:7; or
a PCR primer comprising SEQ ID NO:14 and a PCR primer comprising SEQ ID NO:13.

The invention further provides target-specific primers and oligonucleotide probes for use in the detection of *Mycobacterium spp*., particularly for use in detection of *Mycobacterium spp*. by RT-PCR or NASBA.

In particular, the invention provides primer and probe oligonucleotides comprising the sequences represented as SEQ ID NOs: 1 to 42, 61 to 69 and 74 to 97 in Table 1:

**TABLE 1-Mycobacterium-specific sequences for inclusion in primers and probes.**

| ***Mycobacterium tuberculosis*** | | | |
|---|---|---|---|
| **pncA MT59967** | | | |
| **Primer/probe type** | **Sequence** | **sequence number (SEQ ID NO)** | **nt** |
| NASBA P2/PCR | GAAGCGGCGGACTACCATCA | 1 | 109 |
| NASBA P1/PCR | X₁-CTCGATTGCCGACGTGTCCA | 2 | 254 |
| probe | CATTGCGTCAGCGGTACTCC | 3 | |
| MB probe | X₂-CCCGATCATTGCGTCAGCGGTACTCCATCGGG-X₃ | 4 | |
| MB probe | X₂-GCCCGATCATTGCGTCAGCGGTACTCCATCGGGC-X₃ | 5 | |
| MB probe | X₂-CCGCCGCATTGCGTCAGCGGTACTCCCGGCGG-X₃ | 6 | |
| NASBA P2/PCR | ACCAAGGACTTCCACATCGA | 7 | 139 |
| NASBA P1/PCR | X₁-TACCGACCACATCGACCTCA | 8 | 378 |
| probe | ATTGCGTCAGCGGTACTCCC | 9 | 212 |
| MB probe | X₂-CGTCGTATTGCGTCAGCGGTACTCCCACGACG-X₃ | 10 | 212 |
| MB probe | X₂-CGTCGGATTGCGTCAGCGGTACTCCCCCGACG-X₃ | 11 | 212 |
| MB probe | X₂-AGGCGTATTGCGTCAGCGGTACTCCCACGCCT-X₃ | 12 | 212 |
| NASBA P2/PCR | GTGACCACTTCTCCGGCACA | 13 | 164 |
| NASBA P1/PCR | X₁-GTGTAGGCACCCTTGTAGAA | 14 | 280 |
| probe | GACTATTCCTCGTCGTGGCC | 15 | 187 |
| MB probe | X₂-CGCATGGACTATTCCTCGTCGTGGCCCATGCG-X₃ | 16 | 187 |
| MB probe | X₂-CCAGCGACTATTCCTCGTCGTGGCCGCTGG-X₃ | 17 | 187 |
| MB probe | X₂-TCGCGGACTATTCCTCGTCGTGGCCCGCGA-X₃ | 18 | 187 |

| **sodA AF061030** | | | |
|---|---|---|---|
| NASBA P2/PCR | CTGTTTTGGGCGTCGATCCA | 19 | 95 |
| NASBA P1/PCR | X₁-CAGTTGGCGGGGACGGCTAA | 20 | 201 |
| probe | ATTCCCACTACCACGTCCGG | 21 | 123 |
| MB probe | X₂-CGCATGATTCCCACTACCACGTCCGGCATGCG-X₃ | 22 | 123 |
| MB probe | X₂-CCTGCGATTCCCACTACCACGTCCGGCGCAGG-X₃ | 23 | 123 |
| MB probe | X₂-GCGCTATTCCCACTACCACGTCCGGAGCGC-X₃ | 24 | 123 |
| NASBA P2/PCR | GGCGTCGATCCACTGCGGGA | 25 | 103 |
| NASBA P1/PCR | X₁-GGTGGGCAAGAAGAAGTCGA | 26 | 229 |
| probe | GCCCGCTCCAGCACCTCCTT | 27 | 171 |
| MB probe | X₂-CGCATGGCCCGCTCCAGCACCTCCTTCATGCG-X₃ | 28 | 171 |
| MB probe | X₂-CCGTCGGCCCGCTCCAGCACCTCCTTCGACGG-X₃ | 29 | 171 |
| MB probe | X₂-CACGCGCCCGCTCCAGCACCTCCTTGCGTG-X₃ | 30 | 171 |
| NASBA P2/PCR | CTCCAGCACCTCCTTGGTCA | 31 | 176 |
| NASBA P1/PCR | X₁-CCAGGCTGATACCACGTCTA | 32 | 347 |
| probe | ACTTCTTCTTGCCCACCCAC | 33 | 232 |
| MB probe | X₂-CGCATGACTTCTTCTTGCCCACCCACCATGCG-X₃ | 34 | 232 |
| MB probe | X₂-CCGTCGACTTCTTCTTGCCCACCCACCGACGG-X₃ | 35 | 232 |
| MB probe | X₂-CACGCACTTCTTCTTGCCCACCCACGCGTG-X₃ | 36 | 232 |
| NASBA P2/PCR | CCTCCTTGGTCAATTCGTTA | 37 | 184 |
| NASBA P1/PCR | X₁-AAGTCAACCCTGCTCCATGA | 38 | 437 |
| probe | TTGCCCACCCACCCCATAGA | 39 | 240 |
| MB probe | X₂-CGCATGTTGCCCACCCACCCCATAGACATGCG-X₃ | 40 | 240 |
| MB probe | X₂-CGCTGTTGCCCACCCACCCCATAGACAGCG-X₃ | 41 | 240 |
| MB probe | X₂-CGCATTTGCCCACCCACCCCATAGAATGCG-X₃ | 42 | 240 |
| NASBA P2/PCR | GCCGAATACACCTTGCCAGA | 61 | 545 |
| NASBA P1/PCR | X₁-CGTGGTGCTTGCTGTGGTGA | 62 | 622 |
| PROBE | CAATTCAGCGCCGCCGCCAA | 63 | 593 |
| NASBA P2/PCR | ACCTGGACTGGGACTACGGA | 64 | 564 |
| NASBA P1/PCR | X₁-TCATTGGCGCCCTTTACGTA | 65 | 647 |
| PROBE | AATTCAGCGCCGCCGCCAAC | 66 | 594 |
| NASBA P2/PCR | TGGACTGGGACTACGGAGCA | 67 | 567 |
| NASBA P1/PCR | X₁-CGAGTTTGGCGACGGCGTCA | 68 | 664 |

| PROBE | AAGTTCCGGGCGCAATTCAG | 69 | 581 |
|---|---|---|---|
| ***Mycobacterium kansasii, tuberculosis, bovis, avium, intracellulare, leprae, marinum (+ scrofuraceum, gordonae, szulgai, microti, asiaticum) sod A*** | | | |
| NASBA P2/PCR | GCGCCAAGGAAGATCACTCA | 74 | 693 |
| NASBA P1/PCR | X₁-GGCGACAGGTTCTTCCACCA | 75 | 776 |
| PROBE | GAATCTAGCTTTCAACCTCG | 76 | 733 |
| NASBA P2/PCR | CAAGGAAGATCACTCAGCGA | 77 | 697 |
| NASBA P1/PCR | X₁-GTCACCACCGTTAGGCGACA | 78 | 789 |
| PROBE | ATCTAGCTTTCAACCTCGCC | 79 | 735 |
| NASBA P2/PCR | AGCGATCTTGCTGAACGAAA | 80 | 712 |
| NASBA P1/PCR | X₁-TGGGCTTGTCACCACCGTTA | 81 | 796 |
| PROBE | GGCCACGTCAATCACACCAT | 82 | 755 |

| ***Mycobacterium paratuberculosis*** | | | |
|---|---|---|---|
| NASBA P2/PCR | TTCCAGCTCTACGACCAGCA | 83 | 677 |
| NASBA P1/PCR | X₁-TTGGAAGTGGCGGCGGCGTA | 84 | 839 |
| PROBE | AAGGCGGATTACGTCAAGGC | 85 | 779 |
| NASBA P2/PCR | CTCTACGACCAGCAGGCCAA | 86 | 683 |
| NASBA P1/PCR | X₁-CTGCACGTCCGCCCAGTTGA | 87 | 813 |
| PROBE | AAGAACGTCAAGGCGGATTA | 88 | 770 |
| NASBA P2/PCR | TACGACCAGCAGGCCAACGT | 89 | 686 |
| NASBA P1/PCR | X₁-CACGTCCGCCCAGTTGACCA | 90 | 810 |
| PROBE | GTACAAGAACGTCAAGGCGG | 91 | 766 |
| NASBA P2/PCR | TCGAGCTGCCAGTGTGGTCA | 92 | 988 |
| NASBA P1/PCR | X₁-ACGAGAATGGAAGGGGGACA | 93 | 1072 |
| PROBE | AACAGGGTCGGATCGGCCTA | 94 | 1046 |
| NASBA P2/PCR | CCAGTGTGGTCATTTCCATA | 95 | 996 |
| NASBA P1/PCR | X₁-AGGGCACCACGAGAATGGAA | 96 | 1080 |
| PROBE | GCGCGGAGGTCGAAATGGAA | 97 | 1027 |

### KEY:

X₁ represents a nucleotide sequence comprising a promoter. Oligonucleotides adapted for use as NASBA P1 primers have the general structure "X₁-SEQ", wherein "X₁" represents a nucleotide sequence comprising a promoter and "SEQ" represents a target-specific sequence (SEQ ID NO), as given in the accompanying sequence listing. The inclusion of a promoter sequence is essential in NASBA P1 primers but is not necessary in PCR primers, as discussed below. In a preferred embodiment, X₁ may be a sequence comprising a T7 promoter, most preferably the sequence AAT TCT AAT ACG ACT CAC TAT AGG GAG AAGG.
X₂ and X₃ represent a fluorescent moiety and a quencher moiety capable of substantially or completely quenching the fluorescence from the fluorescent moiety when the two are held together in close proximity.
nt - denotes position in the relevant cDNA sequence.

The invention further provides the oligonucleotides listed in Table 2, these being NASBA P1 primers incorporating a particular promoter sequence and NASBA P2 primers incorporating a sequence for binding of a generic detection probe (see below) for use in the detection of *Mycobacteria spp.* by NASBA. However, it is not intended that the scope of the invention should be limited to these specific molecules:

**TABLE 2**

| **SEQUENCE** | **PRIMER NUMBER** | **SEQ ID NO** |
|---|---|---|
| GATGCAAGGTCGCATATGAGGAAGCGGCGGACTACCATCA | 4-1 | 43 |
| | 4-2 | 44 |
| GATGCAAGGTCGCATATGAGACCAAGGACTTCCACATCGA | 4-7 | 45 |
| | 4-8 | 46 |
| GATGCAAGGTCGCATATGAGGTGACCACTTCTCCGGCACA | 4-13 | 47 |
| | 4-14 | 48 |
| GATGCAAGGTCGCATATGAGGCCGAATACACCTTGCCAGA | 4-85 | 70 |
| | 4-86 | 71 |
| GATGCAAGGTCGCATATGAGTGGACTGGGACTACGGAGCA | 4-97 | 72 |
| | 4-98 | 73 |
| GATGCAAGGTCGCATATGAGCTGTTTTGGGCGTCGATCCA | 4-103 | 49 |
| | 4-104 | 50 |
| GATGCAAGGTCGCATATGAGGGCGTCGATCCACTGCGGGA | 4-109 | 51 |
| | 4-110 | 52 |
| GATGCAAGGTCGCATATGAGCTCCAGCACCTCCTTGGTCA | 4-115 | 53 |
| | 4-116 | 54 |
| GATGCAAGGTCGCATATGAGCCTCCTTGGTCAATTCGTTA | 4-121 | 55 |
| | 4-122 | 56 |

The oligonucleotide primers and probes provided by the invention may be grouped into several types. A first type of oligonucleotides are NASBA P1 primers, which are oligonucleotides of generally approximately 50 bases in length, containing an average of about 20 bases at the 3' end that are complementary to a region of the target mRNA. Oligonucleotides suitable for use as NASBA P1 primers are denoted "NASBA P1/PCR" in Table 1. The 5' ends of the P1 primer oligonucleotides (represented herein in general terms as X₁) comprise a promoter sequence that is recognized by a specific RNA polymerase. Bacteriophage promoters, for example the T7, T3 and SP6 promoters, are preferred for use in the oligonucleotides of the invention, since they provide advantages of high level transcription which is dependent only on binding of the appropriate RNA polymerase. In a preferred embodiment, the 5' terminal sequence of the P1 primer oligonucleotides may comprise the sequence AATTCTAATACGACTCACTATAGGG. This sequence contains a T7 promoter, including the transcription initiation site for T7 RNA polymerase. The *Mycobacterium-*specific sequences denoted in Table 1 as "NASBA P1/PCR" are suitable for use in both NASBA P1 primers and standard PCR primers. When these sequences are used as the basis of NASBA P1 primers they have the general structure X₁-SEQ, wherein X₁ represents a sequence comprising a promoter and SEQ represents the target-specific sequence. The promoter sequence X₁ is essential in a NASBA P1 primer. However, when the same sequences are used as the basis of standard PCR primers it is not necessary to include X₁. The phrase "sequence number" as used in the claims is to be interpreted accordingly.

For the avoidance of doubt, the phrase "a NASBA P1 primer comprising SEQ ID NO:1" is to be interpreted as requiring the presence of an X₁ sequence 5' to the *Mycobacterium* specific sequence listed as SEQ ID NO:1, whereas the phrase "a PCR primer comprising SEQ ID NO:1" refers to any suitable PCR primer comprising the *Mycobacterium* specific sequence, X₁ not being an essential feature of a PCR primer.

A second type of oligonucleotides provided by the invention are NASBA primer 2 oligonucleotides which generally comprise a sequence of approximately 20 bases substantially identical to a region of the target mRNA. The oligonucleotide sequences denoted in Table 1 as "NASBA P2/PCR" are suitable for use in both NASBA P1 primers and standard PCR primers. Oligonucleotides intended for use as NASBA P2 primers may, in a particular but non-limiting embodiment, further comprise a sequence of nucleotides at the 5' end which is unrelated to the target mRNA but which is capable of hybridising to a generic detection probe. The detection probe will preferably be labelled, for example with a fluorescent, luminescent or enzymatic label. In one embodiment the detection probe is labelled with a label that permits detection using ECL™ technology, although it will be appreciated that the invention is in no way limited to this particular method of detection. In a preferred embodiment the 5' end of the primer 2 oligonucleotides may comprise the sequence GATGCAAGGTCGCATATGAG. This sequence is capable of hybridising to a generic ECL™ probe commercially available from Organon Teknika having the following structure:
Ru(bpy)₃²⁺-GAT GCA AGC TCG CAT ATG AG-3'

In a different embodiment the primer 2 oligonucleotide may incorporate 'molecular beacons' technology, which is known in the art and described, for example, in WO 95/13399 by Tyagi and Kramer, Nature Biotechnology. 14: 303-308, 1996, to allow for real-time monitoring of the NASBA reaction.

A third type of oligonucleotide molecules provided by the invention are target-specific probe oligonucleotides (denoted "probe" in Table 1). The probe oligonucleotides generally comprise a sequence of approximately 20-25 bases substantially identical to a region of the target mRNA. The probe oligonucleotides may be used as target-specific hybridisation probes for detection of the products of a NASBA or PCR reaction. In this connection the probe oligonucleotides may be coupled to a solid support, such as paramagnetic beads, to form a capture probe (see below). In a preferred embodiment the 5' end of the probe oligonucleotide may be labelled with biotin. The addition of a biotin label facilitates attachment of the probe to a solid support via a biotin/streptavidin or biotin/avidin linkage.

A fourth type of oligonucleotide molecules provided by the invention (denoted as "MB probe" in Table 1) are target-specific probes incorporating "molecular beacons" technology which is known in the art and described, for example, by Tyagi and Kramer, Nature Biotechnology. 14: 303-308, 1996 and in WO 95/13399. The use of molecular beacons technology allows for real-time monitoring of amplification reactions, such as NASBA or RT-PCR reactions.

The molecular beacons probes generally include, in addition to a target-specific sequence, additional bases which allow formation of a hairpin loop structure, a fluorescent moiety and a quencher moiety, the fluorescent and the quencher moieties being represented herein by the notation X₂ and X₃. As will be appreciated be the skilled reader, the fluorescent and quencher moieties are selected such that the quencher moiety is capable of substantially or completely quenching the fluorescence from the fluorescent moiety when the two moieties are in close proximity, e.g. when the probe is in the hairpin "closed" conformation in the absence of the target sequence. The molecular beacons probes shown in Table 1 incorporate complementary flanking sequences at the 5' and 3' ends, flanking the mycobacteria-specific sequences, which are capable of hybridising to form a stem duplex. The precise nucleotide sequence of these flanking sequences is not material to the invention, except for the requirement that they should be capable of forming a stem duplex when the probe is not bound to a target HPV sequence. Therefore, the skilled reader will appreciate that these sequences may be varied without affecting the function of the molecular beacon probes to a material extent. Any of the sequences denoted "probe" in Table 1 may be used as the basis of a molecular beacons probe may the addition of suitable complementary flanking sequences and quencher and fluorescer moieties.

Many examples of suitable pairs of quencher/fluorescer moieties which may be used in molecular beacon probes are known in the art (see WO 95/13399, Tyagi and Kramer, ibid). A broad range of fluorophores in many different colours made be used, including for example 5-(2'-aminoethyl)aminonaphthalene-1-sulphonic acid (EDANS), fluorescein, FAM and Texas Red (see Tyagi, Bratu and Kramer, 1998, Nature Biotechnology, 16, 49-53. The use of probes labelled with different coloured fluorophores enables "multiplex" detection of two or more different probes in a single reaction vessel. A preferred quencher is 4-(4'-dimethylaminophenylazo)benzoic acid (DABCYL), a non-fluorescent chromophore, which serves as a "universal" quencher for a wide range of fluorophores. The fluorescer and quencher moieties may be covalently attached to the probe in either orientation, either with the fluorescer at or near the 5' end and the quencher at or near the 3' end or vice versa. Protocols for the synthesis of molecular beacon probes are known in the art. A detailed protocol for synthesis is provided in a paper entitled "Molecular Beacons: Hybridization Probes for Detection of Nucleic Acids in Homogenous Solutions" by Sanjay Tyagi et al., Department of Molecular Genetics, Public Health Research Institute, 455 First Avenue, New York, NY 10016, USA, which is available online via the PHRI website (at www.phri.nyu.edu or www.molecular-beacons.org).

Suitable combinations of primer 1 and primer 2 oligonucleotide molecules may be used in the detection of a target mRNA by NASBA. In order to drive a NASBA amplification reaction the primer 1 and primer 2 oligonucleotides must be capable of priming synthesis of a double-stranded DNA from a target region of mRNA. For this to occur the primer 1 and primer 2 oligonucleotides must comprise target-specific sequences which are complementary to regions of the sense and the antisense strand of the target mRNA, respectively.

In the first phase of the NASBA amplification cycle, the so-called "non-cyclic" phase, the primer 1 oligonucleotide anneals to a complementary sequence in the target mRNA and it's 3' end is extended by the action of an RNA-dependent DNA polymerase (e.g. reverse transcriptase) to form a first-strand cDNA synthesis. The RNA strand of the resulting RNA:DNA hybrid is then digested, e.g. by the action of RNaseH, to leave a single stranded DNA. The primer 2 oligonucleotide anneals to a complementary sequence towards the 3' end of this single stranded DNA and it's 3' end is extended (by the action of reverse transcriptase), forming a double stranded DNA. RNA polymerase is then able to transcribe multiple RNA copies from the now transcriptionally active promoter sequence within the double-stranded DNA. This RNA transcript, which is antisense to the original target mRNA, can act as a template for a further round of NASBA reactions, with primer 2 annealing to the RNA and priming synthesis of the first cDNA strand and primer 1 priming synthesis of the second cDNA strand. The general principles of the NASBA reaction are well known in the art (see Compton, J. Nature. 350: 91-92).

The target-specific probe oligonucleotides described herein may also be attached to a solid support, such as magnetic microbeads, and used as "capture probes" to immobilise the product of the NASBA amplification reaction (a single stranded RNA). The target-specific "molecular beacons" probes described herein may be used for real-time monitoring of the NASBA reaction.

The invention will be further understood with reference to the following experimental examples, with reference to the accompanying Figures in which:

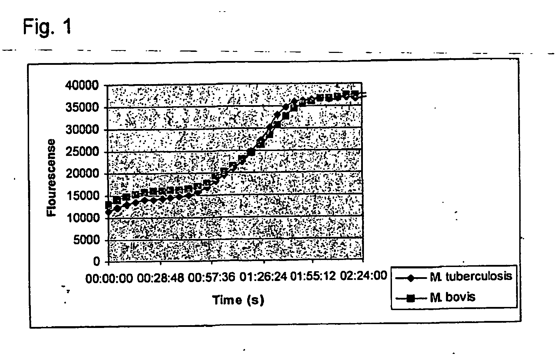

Figure 1 illustrates a time course for amplification of *M. tuberculosis* and *M*. *bovis* sodA mRNA using real-time NASBA amplification with molecular beacons probes. X-axis fluorescence (relative units), y-axis time (seconds).

### Example 1-Induction of bacterial gene expression

### 1) Treatment with oxygen

The specimen containing the slow growing mycobacteria is inoculated into liquid medium. The bacteria are exposed to oxygen gas (bubbling) for 10 and 60 minutes.

### Samples: ,

Due to a variable amount of bacteria collected, the samples are diluted in the liquid medium used for culturing before they are transferred to the lysis buffer. Dilutions -
1:1 and 1:100

### 2) Inoculation in growth media

Place the bacterial sample in medium optimal for growth of the bacterium in question over night at 37°C. Even though bacteria will not multiply to a considerable extent, this will be sufficient for induction of genes for initiation of the NASBA amplification reaction.

### Lysis of bacteria

· Lysis buffer (0.9 ml with low pH) is stored at 2-8°C. Before use, the buffer must be heated to 37°C for 30 minutes so that all crystals are dissolved.
· 100 ml bacterium culture is added to 0.9 ml buffer. Mix well.
· The lysis buffer with the bacteria is incubated at 37°C for 10 minutes.
· Store the lysed bacteria at -70°C or extract nucleic acids immediately.

### 3) Extraction of DNA/RNA from the induced bacterium sample

The following procedure was carried out according to the manual from Nuclisens™, Organon Teknika:
· Add 50 µl silica particles to each sample and incubate for 10 min at room temperature. Mix the tubes every 2nd minute.
· Transfer all the material from the sample in the cartridge for use in the Nuclisens™ Extractor.
· Follow the manual for the method: "Extraction of 1 ml plasma".

### Testing of primers for detection of superoxide dismutase in Mycobacterium spp

2 X reagents sphere
2 X 80 µl diluent
Mix well, then add
28 µl NASBA water
32 µl KCl
(All reagents are from the Nuclisens™ system, supplied by Organon Teknika)
Distribute in 8 tubes, 27.5 µl in each tube. Add primers in the following way. (Primer numbers correspond to the primers listed in Table 2)

| Tube No. | Primers |
|---|---|
| 1 | 1.25 µl 4-85 (SEQ ID NO:70) |
| | 1.25 µl 4-86 (SEQ ID NO:71) |
| 2 | 1.25 µl 4-85 (SEQ ID NO:70) |
| | 1.25 µl 4-98 (SEQ ID NO:73) |
| 3 | 1.25 µl 4-97 (SEQ ID NO:72) |
| | 1.25 µl 4-86 (SEQ ID NO:71) |
| 4 | 1.25 µl 4-97 (SEQ ID NO:72) |
| | 1.25 µl 4-98 (SEQ ID NO:73) |
| | |
| 5 | 1.25 µl 4-115 (SEQ ID NO:53) |
| | 1.25 µl 4-116 (SEQ ID NO:54) |
| 6 | 1.25 µl 4-115 (SEQ ID NO:53) |
| | 1.25 µl 4-122 (SEQ ID NO:56) |
| 7 | 1.25 µl 4-121 (SEQ ID NO:55) |
| | 1.25 µl 4-116 (SEQ ID NO:54) |
| 8 | 1.25 µl 4-121 (SEQ ID NO:55) |
| | 1.25 µl 4-122 (SEQ ID NO:56) |

Samples: *M. tuberculosis* and *M. bovis*
Amplification was performed 41°C for 2.5 hours.
Products were detected on an Agilent Bioanalyser
Results: The primers detect both *M. tuberculosis* and *M. bovis* as expected from their sequence.

### Example 3 Amplification of Mycobacterium tuberculosis from a patient sample.

Samples
1) ATTC bacterium strain grown in culture (control)
2) Patient sample

### Mastermix:

2 reagent sphere
2 X 80 µl reagent sphere diluent

Mixed in one tube, added
23 µl nasbawater
10 µl primer 1 (4-122; SEQ ID NO:56)
10 µl primer 2 (4-115; SEQ ID NO:53)
5 µl molecular beacons (4-125; SEQ ID NO:41)
32 µl KCl

### Samples run:

| | Sample no |
|---|---|
| B | 1a |
| C | 2a |
| F | 1b |
| G | 2b |

Results, increase in signal

| | 1 |
|---|---|
| B | 2.38 |
| C | 1.72 |
| F | 2.17 |
| G | 2.25 |

### Example 4 NASBA amplification with molecular beacons of Mycobacterium tuberculosis

2 X reagents sphere
2 X 80 µl diluent

1) Is added to
   11.5 µl water
   5 µl primer 4-115 (SEQ ID NO:53)
   5 µl primer 4-122 (SEQ ID NO:56)
   16 µl KCl
2) and also added to
   11.5 µl water
   5 µl primer 4-121 (SEQ ID NO:55)
   5 µl primer 4-116 (SEQ ID NO:54)
   16 µl KCl

The two primer mixes are divided into 4 tubes (a total of 8), 29.5 µl in each tube.
Add molecular beacons; 0.63 µl in each tube:
1 a) 4-118 (SEQ ID NO:34)
1 b) 4-119 (SEQ ID NO:35)
1 c) 4-124 (SEQ ID NO:40)
1 d) 4-125 (SEQ ID NO:41)

2 a) 4-118 (SEQ ID NO:34)
2 b) 4-119 (SEQ ID NO:35)
2 c) 4-124 (SEQ ID NO:40)
2 d) 4-125 (SEQ ID NO:41)
2 X enzyme is diluted in 2 X 55 µl diluent
Reagents from NucliSens, OrganonTeknika

### Samples:

Sample 2 and 24 is *M*. *tuberculosis*
Sample 7 is *M. bovis*

| | 1 | | 2 | | 3 | |
|---|---|---|---|---|---|---|
| A | 1a | 2 | 1c | 24 | 2b | 7 |
| B | 1a | 7 | 1d | 2 | 2b | 24 |
| C | 1a | 24 | 1d | 7 | 2c | 2 |
| D | 1b | 2 | 1d | 24 | 2c | 7 |
| E | 1b | 7 | 2a | 2 | 2c | 24 |
| F | 1b | 24 | 2a | 7 | 2d | 2 |
| G | 1c | 2 | 2a | 24 | 2d | 7 |
| H | 1c | 7 | 2b | 2 | 2d | 24 |

Amplification and detection in reader at 41°C for 2.5 timer.
**Results, times increase in signal.**
An increase of 1.5 is regarded as positive

| | 1 | | 2 | | 3 | |
|---|---|---|---|---|---|---|
| A | 1a | 1.94 | 1c | 2.46 | 2b | 1.62 |
| B | 1a | 1.97 | 1d | 2.70 | 2b | 1.64 |
| C | 1a | 1.92 | 1d | 2.46 | 2c | 2.63 |
| D | 1b | 1.63 | 1d | 2.53 | 2c | 2.67 |
| E | 1b | 1.73 | 2a | 1.92 | 2c | 2.55 |
| F | 1b | 1.69 | 2a | 1.95 | 2d | 2.71 |
| G | 1c | 2.63 | 2a | 1.87 | 2d | 2.61 |
| H | 1c | 2.49 | 2b | 1.70 | 2d | 2.52 |

Amplification was detected for all samples - best results detected for mastermix no 1d.

Optimal results detected for the following combination of primers and molecular beacons:
primer2: 4-115 (SEQ ID NO:53)
primer1: 4-122 (SEQ ID NO:56)
molecular beacon: 4-125 (SEQ ID NO:41)

See graphic presentation of amplification in Figure 1

### Example 5 Specificity of Mycobacterium tuberculosis, primers for sodA

### Samples:

| Name (number) |
|---|
| Mycobacterium tuberculosis (3) |
| Mycobacterium bovis (8) |
| Mycobacterium avium-intracellulare (16) |
| Mycobacterium species (22) |
| Mycobacterium gordonae (19) |
| Mycobacterium maloense(21) |
| Mycobacterium xenopi (23) |
| Micrococcus luteus |
| Salmonella gallinarum |
| Staphylococcus aureus |
| Bacillus megaterium |
| Enterovirus |
| water |

### Mastermix:

2 reagent sphere 2 enzyme sphere 2 X 80 µl reagent sphere diluent 2 X 55 µl enzyme diluent

Mix in one tube, then add
23 µl nasba water
10 µl primer 1 (4-122; SEQ ID NO:56)
10 µl primer 2 (4-115; SEQ ID NO:53)
5 µl molecular beacons (4-125; SEQ ID NO:41)
32 µl KCl

### Samples run

| | 1 | 2 | 3 |
|---|---|---|---|
| A | M. tuberculosis | M. gordonae | Salmonella |
| B | M. tuberculosis | M. gordonae | Salmonella |
| C | M. bovis | M. maloense | Staphylococcus |
| D | M. bovis | M. maloense | Staphylococcus |
| E | M. avium-intra. | M. xenopi | Bacillus |
| F | M. avium-intra. | M. xenopi | Bacillus |
| G | - species | Micrococcus | Enterovirus |
| H | - species | Micrococcus | Water |

### Results

Times increase in signal (positive signal: above 1.5)

| | 1 | 2 | 3 |
|---|---|---|---|
| A | 2.77 | 1.09 | 1.09 |
| B | 2.59 | 1.09 | 1.11 |
| C | 2.65 | 1.08 | 1.09 |
| D | 2.63 | 1.07 | 1.10 |
| E | 1.08 | 1.05 | 1.08 |
| F | 1.00 | 1.05 | 1.08 |
| G | 0.98 | 1.07 | 1.09 |
| H | 0.98 | 1.06 | 1.06 |

Conclusion: The primers are specific for *M*. *tuberculosis* or *M. bovis* and will not react with other mycobacteria or related bacteria like *Micrococcus luteus.*

### SEQUENCE LISTING

SEQ ID NO:57 Nucleotide sequence for *M.tuberculosis* sodA
SEQ ID NO:58 Amino acid sequence for *M.tuberculosis* sodA
SEQ ID NO:59 Nucleotide sequence for *M.avium subsp. paratuberculosis* sod
SEQ ID NO:60 Amino acid sequence for *M.avium subsp*. *paratuberculosis* sod
SEQ ID NO:98 Nucleotide sequence for *M.tuberculosis* pncA
SEQ ID NO:99 Amino acid sequence for *M.tuberculosis* pncA

## Claims

1. A method for detecting the presence of a microorganism in a test sample which comprises exposing the test sample to an inducer which is capable of inducing expression of at least one gene in the microorganism and testing for the presence of mRNA transcribed from said gene which is induced by exposure to the said inducer wherein the testing for the presence of said mRNA transcribed from said gene which is induced by exposure to the said inducer comprises performing an amplification reaction to amplify the whole or a portion of the mRNA and detecting specific products of the amplification reaction, wherein the inducer is a chemical agent.

2. A method according to claim 1 wherein the amplification reaction is RT-PCR or nucleic acid sequence-based amplification.

3. A method according to any one of claims 1 or 2, wherein the inducer is oxygen or hydrogen peroxide and the gene which is induced by exposure to the inducer is a gene encoding superoxide dismutase.

4. A method according to claim 3 wherein the step of testing for the presence of mRNA transcribed from the gene encoding superoxide dismutase comprises:
(a) assembling a reaction mixture comprising a NASBA P1 and NASBA P2 primer pair suitable for use in amplification of a region of the said mRNA by NASBA, an RNA directed DNA polymerase, a ribonuclease that hydrolyses the RNA strand of an RNA-DNA hybrid without hydrolysing single or double stranded RNA or DNA, an RNA polymerise that recognises a promoter sequence present in the NASBA P1 primer and ribonucleoside and deoxyribonucleoside triphosphates;
(b) incubating said reaction medium with a preparation of nucleic acid isolated from a test sample suspected of containing the microorganism under reaction conditions which permit a NASBA amplification reaction; and
(c) detecting and/or quantitatively measuring any specific product of the NASBA amplification reaction.

5. A method according to claim 4 for use in the detection of *Mycobacterium Spp.* wherein the NASBA P1 and NASBA P2 primer pair is one of the following:
a NASBA P1 primer comprising SEQ ID NO:20 and a NASBA P2 primer comprising SEQ ID NO:19;
a NASBA P1 primer comprising SEQ ID NO:26 and a NASBA P2 primer comprising SEQ ID NO:25;
a NASBA P1 primer comprising SEQ ID NO:32 and a NASBA P2 primer comprising SEQ ID NO:31;
a NASBA P1 primer comprising SEQ ID NO:38 and a NASBA P2 primer comprising SEQ ID NO:37;
a NASBA P1 primer comprising SEQ ID NO:61 and a NASBA P2 primer comprising SEQ ID NO:62;
a NASBA P1 primer comprising SEQ ID NO:64 and a NASBA P2 primer comprising SEQ ID NO:65;
a NASBA P1 primer comprising SEQ ID NO:71 and a NASBA P2 primer comprising SEQ ID NO:61, preferably a primer comprising SEQ ID NO:70;
a primer comprising SEQ ID NO:50 and a NASBA P2 primer comprising SEQ ID NO:19, preferably a primer comprising SEQ ID NO:49; or
a primer comprising SEQ ID NO:56 and a NASBA P2 primer comprising SEQ ID NO:31, preferably a primer comprising SEQ ID NO:53.

6. A method according to claim 3 wherein the step of testing for the presence of mRNA transcribed from the gene encoding superoxide dismutase comprises amplifying a portion of the mRNA by RT-PCR.

7. A method according to claim 6 for use in the detection of *Mycobacterium Spp.* wherein amplification of a portion of the mRNA by RT-PCR is carried out using one of the following primer pairs:
a PCR primer comprising SEQ ID NO:20 and PCR primer comprising SEQ ID NO:19;
a PCR primer comprising SEQ ID NO:26 and a PCR primer comprising SEQ ID NO:25;
a PCR primer comprising SEQ ID NO:32 and a PCR primer comprising SEQ ID NO:31;
a PCR primers comprising SEQ ID NO:38 and a PCR primer comprising SEQ ID NO:37;
a PCR primer comprising SEQ ID NO:61 and a PCR primer comprising SEQ ID NO:62; or
a PCR primer comprising SEQ ID NO:64 and a PCR primer comprising SEQ ID NO:65.

8. A method according to any one of claims 3 to 5 wherein the inducer is isopropyl-β-D-thiogalactopyranoside or pyrazinamide and the gene which is induced by exposure to the inducer is a *pncA* gene.

9. A method according to claim 8 wherein the step of testing for the presence of mRNA transcribed from the *pncA* gene comprises:
(a) assembling a reaction mixture comprising a NASBA P1 and NASBA P2 primer pair suitable for use in amplification of a region of the said mRNA by NASBA, an RNA directed DNA polymerase, a ribonuclease that hydrolyses the RNA strand of an RNA-DNA hybrid without hydrolysing single or double stranded RNA or DNA, an RNA polymerase that recognises a promoter sequence present in the NASBA P1 primer and ribonucleoside and deoxyribonucleoside triphosphates;
(b) incubating said reaction medium with a preparation of nucleic acid isolated from a test sample suspected of containing the microorganism under reaction conditions which permit a NASBA amplification reaction; and
(c) detecting and/or quantitatively measuring any specific product of the NASBA amplification reaction.

10. A method according to claim 9 for use in detection of Mycobacterium *tuberculosis* wherein the NASBA P1 and NASBA P2 primer pair is one of the following:
a NASBA P1 primer comprising SEQ ID NO:2 and a NASBA P2 primer comprising SEQ ID NO:1;
a NASBA P1 primer comprising SEQ ID NO:8 and a NASBA P2 primer comprising SEQ ID NO:7;
a NASBA P1 primer comprising SEQ ID NO:14 and a NASBA P2 primer comprising SEQ ID NO:13;
a primer comprising SEQ ID NO:44 and a NASBA P2 primer comprising SEQ ID NO:1, preferably a primer comprising SEQ ID:43;
a primer comprising SEQ ID NO:46 and a NASBA P2 primer comprising SEQ ID NO:7, preferably a primer comprising SEQ ID NO: 45; or
a primer comprising SEQ ID NO:48 and a NASBA P2 primer comprising SEQ ID NO:13, .

11. A method according to claim 8 wherein the step of testing for the presence of mRNA transcribed from the *pncA* gene comprises amplifying a portion of the mRNA by RT-PCR.

12. A method according to claim 11 for use in detection of Mycobacterium tuberculosis wherein amplification of a portion of the mRNA by RT-PCR is carried out using one of the following primer pairs:
a PCR primer comprising SEQ ID NO:2. and a PCR primer comprising SEQ ID NO:1;
a PCR primer comprising SEQ ID NO:8 and a PCR primer comprising SEQ ID NO:7; or
a PCR primer comprising SEQ ID NO:14 and a PCR primer comprising SEQ ID NO:13.

## Patentansprüche

1. Verfahren zum Nachweis des Vorliegens eines Mikroorganismus in einer Testprobe, das umfasst:
- Inkontaktbringen der Testprobe mit einem Induzierungsmittel, das befähigt ist, die Expression mindestens eines Gens in dem Mikroorganismus zu induzieren, und
- Testen auf das Vorliegen von mRNA, die von dem Gen transkribiert wurde, das durch das Inkontaktbringen mit dem Induzierungsmittel induziert wurde,
wobei das Testen auf das Vorliegen der mRNA, die von diesem Gen transkribiert wurde, das durch das Inkontaktbringen mit dem Induzierungsmittel induziert wurde, die Durchführung einer Amplifizierungsreaktion zur Amplifizierung der ganzen mRNA oder eines Teils davon und den Nachweis spezifischer Produkte der Amplifizierungsreaktion umfasst, wobei das Induzierungsmittel ein chemisches Mittel ist.

2. Verfahren nach Anspruch 1, bei dem die Amplifizierungsreaktion eine RT-PCR oder eine auf der Nucleinsäuresequenz beruhende Amplifizierung ist.

3. Verfahren nach Anspruch 1 oder 2, bei dem das Induzierungsmittel Sauerstoff oder Wasserstoffperoxid ist und das Gen, das durch Inkontaktbringen mit dem Induzierungsmittel induziert wird, ein Gen ist, das Superoxiddismutase codiert.

4. Verfahren nach Anspruch 3, bei dem der Schritt des Testens auf das Vorliegen von mRNA, die von dem Gen transkribiert wurde, das Superoxiddismutase codiert, umfasst:
(a) Herstellung eines Reaktionsgemisches, das enthält:
ein NASBA P1-NASBA P2-Primerpaar, das zur Verwendung bei der Amplifizierung einer Region der mRNA durch NASBA geeignet ist, eine auf RNA gerichtete DNA-Polymerase, eine Ribonuclease, die den RNA-Strang eines RNA-DNA-Hybrids hydrolysiert, ohne einzelsträngige oder doppelsträngige RNA oder DNA zu hydrolysieren, eine RNA-Polymerase, die eine Promotorsequenz erkennt, die im NASBA P1-Primer vorliegt, und Ribonucleosidtriphosphat und Desoxyribonucleosidtriphosphat;
(b) Inkubation des Reaktionsmediums mit einer Präparation von Nucleinsäure, die aus einer Testprobe isoliert wurde, bei welcher der Verdacht besteht, dass sie den Mikroorganismus enthält, unter Reaktionsbedingungen, die eine NASBA-Amplifizierungsreaktion erlauben,
und
(c) Nachweis und/oder quantitative Messung eines spezifischen Produkts der NASBA-Amplifizierungsreaktion.

5. Verfahren nach Anspruch 4 zur Anwendung beim Nachweis von *Mycobacterium spp.,* bei dem das NASBA P1-NASBA P2-Primerpaar eines der folgenden Primerpaare ist:
ein NASBA P1-Primer, der SEQ ID NO:20 enthält, und ein NASBA P2-Primer, der SEQ ID NO:19 enthält;
ein NASBA P1-Primer, der SEQ ID NO:26 enthält, und ein NASBA P2-Primer, der SEQ ID NO:25 enthält;
ein NASBA P1-Primer, der SEQ ID NO:32 enthält, und ein NASBA P2-Primer, der SEQ ID NO:31 enthält;
ein NASBA P1-Primer, der SEQ ID NO:38 enthält, und ein NASBA P2-Primer, der SEQ ID NO:37 enthält;
ein NASBA P1-Primer, der SEQ ID NO:61 enthält, und ein NASBA P2-Primer, der SEQ ID NO:62 enthält;
ein NASBA P1-Primer, der SEQ ID NO:64 enthält, und ein NASBA P2-Primer, der SEQ ID NO:65 enthält;
ein NASBA P1-Primer, der SEQ ID NO:71 enthält, und ein NASBA P2-Primer, der SEQ ID NO:61 enthält, vorzugsweise ein Primer, der SEQ ID NO:70 enthält;
ein Primer, der SEQ ID NO:50 enthält, und ein NASBA P2-Primer, der SEQ ID NO:19 enthält, vorzugsweise ein Primer, der SEQ ID NO:49 enthält, oder
ein Primer, der SEQ ID NO:56 enthält, und ein NASBA P2-Primer, der SEQ ID NO:31 enthält, vorzugsweise ein Primer, der SEQ ID NO:53 enthält.

6. Verfahren nach Anspruch 3, bei dem der Schritt des Testens auf das Vorliegen von mRNA, die von dem Gen transkribiert wurde, das Superoxiddismutase codiert, die Amplifizierung eines Bereichs der mRNA durch RT-PCR umfasst.

7. Verfahren nach Anspruch 6 zur Anwendung beim Nachweis von *Mycobacterium spp.,* bei dem die Amplifizierung eines Bereichs der mRNA durch RT-PCR unter Verwendung eines der folgenden Primerpaare durchgeführt wird:
eines PCR-Primers, der SEQ ID NO:20 enthält, und eines PCR-Primers, der SEQ ID NO:19 enthält;
eines PCR-Primers, der SEQ ID NO:26 enthält, und eines PCR-Primers, der SEQ ID NO:25 enthält;
eines PCR-Primers, der SEQ ID NO:32 enthält, und eines PCR-Primers, der SEQ ID NO:31 enthält;
eines PCR-Primers, der SEQ ID NO:38 enthält, und eines PCR-Primers, der SEQ ID NO:37 enthält;
eines PCR-Primers, der SEQ ID NO:61 enthält, und eines PCR-Primers, der SEQ ID NO:62 enthält;
eines PCR-Primers, der SEQ ID NO:64 enthält, und eines PCR-Primers, der SEQ ID NO:65 enthält.

8. Verfahren nach einem der Ansprüche 3 bis 5, bei dem das Induzierungsmittel Isopropyl-β-D-thiogalactopyranosid oder Pyrazinamid ist und das Gen, das durch Inkontaktbringen mit dem Induzierungsmittel induziert wird, ein *pncA*-Gen ist.

9. Verfahren nach Anspruch 8, bei dem der Schritt des Testens auf das Vorliegen von mRNA, die von dem *pncA*-Gen transkribiert wurde, umfasst:
(a) Herstellung eines Reaktionsgemisches, das enthält:
ein NASBA P1-NASBA P2-Primerpaar, das zur Verwendung bei der Amplifizierung einer Region der mRNA durch NASBA geeignet ist, eine auf RNA gerichtete DNA-Polymerase, eine Ribonuclease, die den RNA-Strang eines RNA-DNA-Hybrids hydrolysiert, ohne einzelsträngige oder doppelsträngige RNA oder DNA zu hydrolysieren, eine RNA-Polymerase, die eine Promotorsequenz erkennt, die im NASBA P1-Primer vorliegt, und Ribonucleosidtriphosphat und Desoxyribonucleosidtriphosphat;
(b) Inkubation des Reaktionsmediums mit einer Präparation von Nucleinsäure, die aus einer Testprobe isoliert wurde, bei welcher der Verdacht besteht, dass sie den Mikroorganismus enthält, unter Reaktionsbedingungen, die eine NASBA-Amplifizierungsreaktion erlauben,
und
(c) Nachweis und/oder quantitative Messung eines spezifischen Produkts der NASBA-Amplifizierungsreaktion.

10. Verfahren nach Anspruch 9 zur Anwendung beim Nachweis von *Mycobacterium tuberculosis,* bei dem das NASBA P1-NASBA P2-Primerpaar eines der folgenden Primerpaare ist:
ein NASBA P1-Primer, der SEQ ID NO:2 enthält, und ein NASBA P2-Primer, der SEQ ID NO:1 enthält;
ein NASBA P1-Primer, der SEQ ID NO:8 enthält, und ein NASBA P2-Primer, der SEQ ID NO:7 enthält;
ein NASBA P1-Primer, der SEQ ID NO:14 enthält, und ein NASBA P2-Primer, der SEQ ID NO:13 enthält;
ein Primer, der SEQ ID NO:44 enthält, und ein NASBA P2-Primer, der SEQ ID NO:1 enthält, vorzugsweise ein Primer, der SEQ ID NO:43 enthält;
ein Primer, der SEQ ID NO:46 enthält, und ein NASBA P2-Primer, der SEQ ID NO:7 enthält, vorzugsweise ein Primer, der SEQ ID NO:45 enthält, oder
ein Primer, der SEQ ID NO:48 enthält, und ein NASBA P2-Primer, der SEQ ID NO:13 enthält.

11. Verfahren nach Anspruch 8, bei dem der Schritt des Testens auf das Vorliegen von mRNA, die von dem pncA-Gen transkribiert wurde, die Amplifizierung eines Bereichs der mRNA durch RT-PCR umfasst.

12. Verfahren nach Anspruch 11 zur Anwendung beim Nachweis von *Mycobacterium tuberculosis*, bei dem die Amplifizierung eines Bereichs der mRNA durch RT-PCR unter Verwendung eines der folgenden Primerpaare durchgeführt wird:
- eines PCR-Primers, der SEQ ID NO:2 enthält, und eines PCR-Primers, der SEQ ID NO:1 enthält;
- eines PCR-Primers, der SEQ ID NO:8 enthält, und eines PCR-Primers, der SEQ ID NO:7 enthält, oder
- eines PCR-Primers, der SEQ ID NO:14 enthält, und eines PCR-Primers, der SEQ ID NO:13 enthält.

## Revendications

1. Méthode pour détecter la présence d'un microorganisme dans un échantillon test qui comprend l'exposition de l'échantillon test à un inducteur qui est apte à induire l'expression d'au moins un gène dans le microorganisme et le test en vue de la présence de ARNm transcrit dudit gène qui est induit par l'exposition audit inducteur, où le test en vue de la présence dudit ARNm transcrit dudit gène qui est induit par l'exposition audit inducteur comprend l'exécution d'une réaction d'amplification pour amplifier l'ensemble ou une partie du ARNm et la détection de produits spécifiques de la réaction d'amplification, dans laquelle l'inducteur est un agent chimique.

2. Méthode selon la revendication 1, où la réaction d'amplification est une amplification de RT-PCR ou basée sur une séquence d'acides nucléiques.

3. Méthode selon l'une quelconque des revendications 1 ou 2, dans laquelle l'inducteur est l'oxygène ou le peroxyde d'hydrogène, et le gène qui est induit par l'exposition à l'inducteur est un gène codant pour une superoxyde dismutase.

4. Méthode selon la revendication 3, dans laquelle l'étape de test en vue de la présence de ARNm transcrit du gène codant pour la superoxyde dismutase comprend:
(a) assembler un mélange de réaction comprenant une paire d'amorces NASBA P1 et NASBA P2 apte à être utilisée dans l'amplification d'une région dudit ARNm par NASBA, une ADN polymérase dirigée ARN, une ribonucléase qui hydrolyse le brin ARN d'un hybride ARN-ADN sans hydrolyser l'ARN ou l'ADN à brin simple ou double, une ARN polymérase qui reconnaît une séquence promoteur présente dans l'amorce NASBA P1 et des triphosphates de ribonucléoside et désoxyribonucléoside;
(b) incuber ledit milieu de réaction avec une préparation d'acide nucléique isolée d'un échantillon test supposé contenir le microorganisme sous des conditions de réaction qui permettent une réaction d'amplification NASBA; et
(c) détecter et/ou mesurer quantitativement tout produit spécifique de la réaction d'amplification NASBA.

5. Méthode selon la revendication 4 pour utilisation dans la détection de *Mycobacterium Spp*. dans laquelle la paire d'amorces de NASBA P1 et NASBA P2 est une des suivantes:
une amorce NASBA P1 comprenant la SEQ ID NO:20 et une amorce NASBA P2 comprenant la SEQ ID NO:19;
une amorce NASBA P1 comprenant la SEQ ID NO:26 et une amorce NASBA P2 comprenant la SEQ ID NO:25;
une amorce NASBA P1 comprenant la SEQ ID NO:32 et une amorce NASBA P2 comprenant la SEQ ID NO:31;
une amorce NASBA P1 comprenant la SEQ ID NO:38 et une amorce NASBA P2 comprenant la SEQ ID NO:37;
une amorce NASBA P1 comprenant la SEQ ID NO:61 et une amorce NASBA P2 comprenant la SEQ ID NO:62;
une amorce NASBA P1 comprenant la SEQ ID NO:64 et une amorce NASBA P2 comprenant la SEQ ID NO:65;
une amorce NASBA P1 comprenant la SEQ ID NO:61, de préférence une amorce NASBA P2 comprenant la SEQ ID NO:70;
une amorce comprenant la SEQ ID NO:50 et une amorce NASBA P2 comprenant la SEQ ID NO:19, de préférence une amorce comprenant la SEQ ID NO:49;
une amorce comprenant la SEQ ID NO:56 et une amorce NASBA P2 comprenant la SEQ ID NO:31, de préférence une amorce comprenant la SEQ ID NO:53.

6. Méthode selon la revendication 3, dans laquelle l'étape consistant à tester la présence de ARNm transcrit du gène codant pour une superoxyde dismutase comprend l'amplification d'une portion de l'ARNm par RT-PCR.

7. Méthode selon la revendication 6 pour utilisation dans la détection de *Mycobacterium Spp*. dans laquelle l'amplification d'une portion de la ARNm par RT-PCR est exécutée en utilisant l'une des paires d'amorces suivantes:
une amorce PCR comprenant la SEQ ID NO:20 et une amorce PCR comprenant la SEQ ID NO:19;
une amorce PCR comprenant la SEQ ID NO:26 et une amorce PCR comprenant la SEQ ID NO:25;
une amorce PCR comprenant la SEQ ID NO:32 et une amorce PCR comprenant la SEQ ID NO:31.
une amorce PCR comprenant la SEQ ID NO:38 et une amorce PCR comprenant la SEQ ID NO:37;
une amorce PCR comprenant la SEQ ID NO:61 et une amorce PCR comprenant la SEQ ID NO:62; ou
une amorce PCR comprenant la SEQ ID NO:64 et une amorce PCR comprenant la SEQ ID NO:65.

8. Méthode selon l'une quelconque des revendications 3 à 5, dans laquelle l'inducteur est l'isopropyl-β-D-thiogalactopyranoside ou pyrazinamide, et le gène qui est induit par l'exposition à l'inducteur est un gène *pncA*.

9. Méthode selon la revendication 8, dans laquelle l'étape de test en vue de la présence de ARNm transcrit du gène *pncA* comprend:
(a) assembler un mélange de réaction comprenant une paire d'amorces de NASBA P1 et de NASBA P2 apte à être utilisée dans l'amplification d'une région dudit ARNm par NASBA, une ADN polymérase dirigée ARN, une ribonucléase qui hydrolyse le brin ARN d'un hybride ARN-ADN sans hydrolyser l'ARN ou l'ADN à brin simple ou double, une ARN polymérase qui reconnaît une séquence promoteur présente dans l'amorce de NASBA P1 et des triphosphates de ribonucléoside et désoxyribonucléoside;
(b) incuber ledit milieu de réaction avec une préparation d'acide nucléique isolée d'un échantillon test supposé contenir le microorganisme sous des conditions de réaction qui permettent une réaction d'amplification de NASBA; et
(c) détecter et/ou mesurer quantitativement tout produit spécifique de la réaction d'amplification de NASBA.

10. Méthode selon la revendication 9 pour utilisation dans la détection de *Mycobacterium tuberculosis*, dans laquelle la paire d'amorces de NASBA P1 et NASBA P2 est une des suivantes:
une amorce de NASBA P1 comprenant la SEQ ID NO:2 et une amorce de NASBA P2 comprenant la SEQ ID NO:1;
une amorce de NASBA P1 comprenant la SEQ ID NO: 8 et une amorce de NASBA P2 comprenant la SEQ ID NO:7;
une amorce NASBA P1 comprenant la SEQ ID NO:14 et une amorce de NASBA P2 comprenant la SEQ ID NO:13;
une amorce comprenant la SEQ ID NO:44 et une amorce de NASBA P2 comprenant la SEQ ID NO:1, de préférence comprenant la SEQ ID NO:43;
une amorce comprenant la SEQ ID NO:46 et une amorce de NASBA P2 comprenant la SEQ ID NO:7, de préférence une amorce comprenant la SEQ ID NO: 45;
une amorce comprenant la SEQ ID NO:48 et une amorce de NASBA P2 comprenant la SEQ ID NO:13.

11. Méthode selon la revendication 8, dans laquelle l'étape de test en vue de la présence d'ARNm transcrit du gène *pncA* comprend l'amplification d'une portion du ARNm par RT-PCR.

12. Méthode selon la revendication 11 pour utilisation dans la détection de *Mycobacterium tuberculosis*, dans laquelle l'amplification d'une portion de l'ARNm par RT-PCR est exécutée en utilisant l'une des paires d'amorces suivantes:
une amorce PCR comprenant la SEQ ID NO:2 et une amorce PCR comprenant la SEQ ID NO:1;
une amorce PCR comprenant la SEQ ID NO:8 et une amorce PCR comprenant la SEQ ID NO:7; ou
une amorce PCR comprenant la SEQ ID NO:14 et une amorce PCR comprenant la SEQ ID NO:13.
